# EUROPEAN PATENT APPLICATION

(11) **EP 4 335 452 A1**
(43) Date of publication of application: **13.03.2024**
(21) Application number: 22771766.7
(22) Date of filing: 16.03.2022
(51) Int. Cl.: A61K 38/08, A61K 31/506, A61K 45/06, A61P 35/00, A61P 35/02

(54) **PHARMACEUTICAL COMPOSITION FOR ENHANCING ANTICANCER EFFECT OF ANTICANCER DRUG**

(30) Priority: 17.03.2021 KR 20210034947
(71) Applicant: L-Base Co., Ltd., Seoul 04778 (KR)
(72) Inventor: JEON, Do Yong, Seoul 01408 (KR); MOON, Chang Hoon, Ulsan 44202 (KR); SHIN, Hyun Hee, Ulsan 44202 (KR); LEE, Hye Rim, Busan 47803 (KR); KIM, Jeong Min, Seoul 05364 (KR); LEE, Jee Young, Gunpo-si Gyeonggi-do 15826 (KR)
(74) Representative: Dehns
(86) International application number: PCT/KR2022/003692
(87) International publication number: WO 2022/197100

(57) **Abstract**

The present invention relates to a compound for enhancing anticancer effect of targeted anticancer drugs and chemotherapeutic agents currently in use and, more particularly, to an analog compound of the oligopeptide AQTGTGKT and a composition comprising the same, wherein the analog compound exhibits an excellent anticancer effect without any side effects. When the compound according to the present invention is mixed with an anticancer drug for combined treatment, as compared with a control group and each of single treatment groups, a significant synergistic effect in inhibitory effects on cancer growth may be provided. Furthermore, since a low-dose anticancer drug can exhibit an excellent combined anticancer effect, side effects including damage to functions and activities that appear in normal tissues, bone marrow dysfunction, gastrointestinal disturbance, alopecia, anticancer drug resistance, and the like, can be minimized. In addition, the oligopeptide has a smaller molecular weight than that of an antibody, and thus an immune reaction of the oligopeptide is of less concern, and the oligopeptide has the advantage of being easy to permeate into tissue, expecting use in combination with an anticancer drug for various types of cancer.

## Description

### [Technical Field]

The present invention relates to a compound that may exhibit synergistic effects through co-administration with anticancer drugs such as targeted anticancer drugs and chemotherapeutic agents for cancer treatment and a pharmaceutical composition comprising the compound and anticancer drugs, and the like.

The present invention claims priority based on Korean Patent Application No. 10-2021-0034947 filed on March 17, 2021 and Korean Patent Application No. 10-2022-0032699 filed on March 16, 2022. The contents disclosed in the specifications and drawings of these applications are incorporated herein by reference.

### [Background Art]

Despite the development of early cancer diagnostic methods and the continuous development of new anticancer therapies improving the anticancer effect, cancer remains a serious disease competing for the first and second cause of death in South Korea. The majority of anticancer drugs currently used are chemotherapeutic agents, which have various pharmacological effects depending on the type of cancer and various side effects due to toxicity, which are pointed out as problems in cancer treatment. Thus, continuous development of targeted therapeutic agents with clear anticancer mechanisms is needed to overcome these limitations of the chemotherapy system.

Existing anticancer drugs penetrate not only cancer cells but also normal cells, causing damage to the function and activity of the normal cells. This may lead to side effects such as bone marrow dysfunction, gastrointestinal disturbance, alopecia, and the problem of multi-drug resistance due to long-term chemotherapy, representing significant problems in cancer treatment. Therefore, active development is underway for co-administered drugs that may maximize the efficiency of the anticancer drugs while minimizing side effects as a means to solve these serious problems of the existing anticancer drugs.

Particularly, co-administration for cancer treatment, which attacks cancer cells through multiple means, is widely used in cancer treatment. KR No. 10-2014-0097607 has disclosed a pharmaceutical composition for co-administration for cancer treatment. The co-administration may enhance the efficacy of the anticancer drugs while reducing the amount of administered anticancer drugs, thereby minimizing the toxicity and side effects of the anticancer drugs, and is useful even when resistance to the anticancer drugs has emerged. Despite numerous effective co-administration therapies have been confirmed over the past decades, given the continually rising number of people dying from cancer each year, it is important to develop effective co-administration therapies.

### [Disclosure]

### [Technical Problem]

The present invention was conceived to solve the problems in the related art as described above, and the present invention was completed by confirming that a significant increase in anticancer activity was evident when the anticancer active peptide AQTGTGKT (alanine-glutamine-threonine-glycine-threonine-glycine-lysine-threonine) and its amidated analog compounds are co-administered with existing anticancer drugs.

Therefore, the present invention is directed to providing a pharmaceutical composition for preventing or treating cancer, which comprises the compound and the anticancer drugs according to the present invention as active ingredients.

The present invention is also directed to providing a kit for preventing or treating cancer, which comprises the compound and the anticancer drugs according to the present invention as active ingredients.

The present invention is also directed to providing a pharmaceutical composition for enhancing the anticancer effect of anticancer drugs.

However, the technical problems that the present invention aims to solve are not limited to the problems mentioned above, and other problems not mentioned will be clearly understood by those of ordinary skill in the art from the description below.

### [Technical Solution]

To achieve the purposes of the present invention as described above, the present invention provides (i) a compound represented by the following General Formula; and (ii) a pharmaceutical composition for preventing or treating cancer, comprising an anticancer drug as an active ingredient:

[General Formula] X-AQTGTGKT

(In the General Formula, A represents alanine, Q represents glutamine, T represents threonine, G represents glycine, K represents lysine, and

X is absent; or at least one selected from the group consisting of

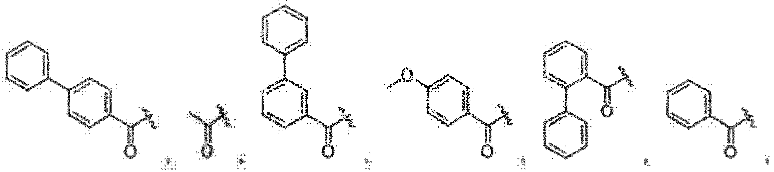

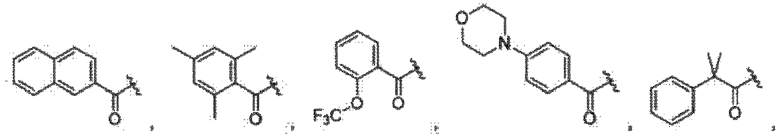

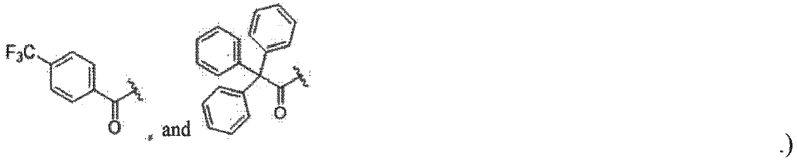

In one embodiment of the present invention, the X may be absent; or at least one selected from the group consisting of 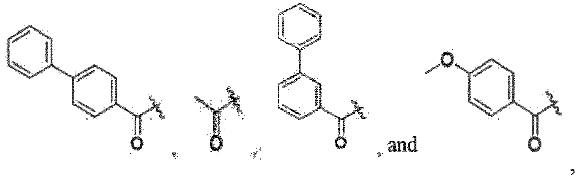 but is not limited thereto.

In another embodiment of the present invention, the anticancer drug may be at least one selected from the group consisting of targeted anticancer drugs and chemotherapeutic agents, but is not limited thereto.

In yet another embodiment of the present invention, the targeted anticancer drugs may be at least one selected from the group consisting of tyrosine kinase inhibitor, PARP inhibitor, angiogenesis inhibitor, cyclin-dependent kinases 4/6 inhibitor (CDK4/6 inhibitor), hormonal therapy drug, and antibody-drug conjugate, but is not limited thereto.

In yet another embodiment of the present invention, the tyrosine kinase inhibitor may be a target drug for at least one selected from the group consisting of epidermal growth factor receptor (EGFR), anaplastic lymphoma kinase (ALK), ROS Proto-Oncogene 1 (ROS1), B-Raf Proto-Oncogene (BRAF), human epidermal growth factor receptor 2 (HER2), Ret Proto-Oncogene (RET), Neurotrophic Receptor Tyrosine Kinase 1 (NTRK1), Mesenchymal-Epithelial Transition factor (MET), and Neuregulin 1 (NRG1), but is not limited thereto.

In yet another embodiment of the present invention, the tyrosine kinase inhibitor may be at least one selected from the group consisting of Osimertinib, Afatinib, Brigatinib, Dasatinib, Dacomitinib, Erlotinib, Gefitinib, Lapatinib, Neratinib, Vandetanib, Icotinib, Varitinib, Tesevatinib, Canertinib, Naquotinib, Pelitinib, Poziotinib, Rociletinib, Nazartinib, Allitinib (ALS-1306), Pyrotinib, Tyrphostin, Crizotinib, Ceritinib, Entrectinib, Dabrafenib, Trametinib, Alectinib, Lorlatinib, Larotectinib, Lasertinib, Olmutinib, AG 1478, CUDC-101, MTKi-327 (JNJ-26483327), CL-387785 (EKI-785), CNX-2006, PD168393, TAK285, WZ4002, and AV-412 (MP-412), but is not limited thereto.

In yet another embodiment of the present invention, the PARP inhibitor may be at least one selected from the group consisting of Olaparib, Rucaparib, Talazoparib, Veliparib, and Niraparib, but is not limited thereto.

In yet another embodiment of the present invention, the CDK4/6 inhibitor may be at least one selected from the group consisting of Trilaciclib, Palbociclib, Ribociclib, and Abemaciclib, but is not limited thereto.

In yet another embodiment of the present invention, the hormonal therapy drug may be at least one selected from the group consisting of Tamoxifen, Toremifene, Fulvestrant, Goserelin, Leuprolide, Anastrozole, Letrozole, and Exemestane, but is not limited thereto.

In yet another embodiment of the present invention, the antibody-drug conjugate may be at least one selected from the group consisting of Sacituzumab govitecan and Ladiratuzumab, but is not limited thereto.

In yet another embodiment of the present invention, the targeted anticancer drug may be at least one selected from the group consisting of Daratumumab, Trastuzumab, and Rituximab, but is not limited thereto.

In yet another embodiment of the present invention, the chemotherapeutic agent may be at least one selected from the group consisting of Alimta, Oxaliplatin, Pemetrexed, Cisplatin, Gemcitabine, Carboplatin, Fluorouracil (5-FU), Cyclophosphamide, Paclitaxel, Vincristine, Etoposide, and Doxorubicin, but is not limited thereto.

In yet another embodiment of the present invention, the compound may enhance the anticancer effect of the anticancer drug and reduce side effects, but is not limited thereto.

In yet another embodiment of the present invention, the composition may be a mixture form in which the compound and the anticancer drug are mixed, but is not limited thereto.

In yet another embodiment of the present invention, the composition may be a form in which the compound and the anticancer drug are each formulated and administered simultaneously or sequentially, but is not limited thereto.

In yet another embodiment of the present invention, the anticancer drug may be included at a concentration of 0.1 to 10 µM relative to the total composition, but is not limited thereto.

In yet another embodiment of the present invention, the compound represented by the General Formula may be included at a concentration of 1 to 50 µM relative to the total composition, but is not limited thereto.

In yet another embodiment of the present invention, the anticancer drug is a targeted anticancer drug, and X in the compound represented by the General Formula may be absent; or at least one selected from the group consisting of 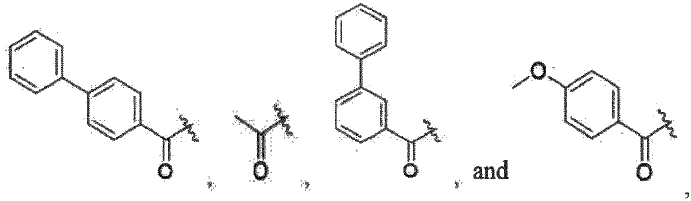 but is not limited thereto.

In yet another embodiment of the present invention, the targeted anticancer drugs and the compound may be included at a molarity ratio of 1:1 to 1:500, but is not limited thereto.

In yet another embodiment of the present invention, the anticancer drug is a chemotherapeutic agent, and X in the compound represented by the General Formula may be at least one selected from the group consisting of 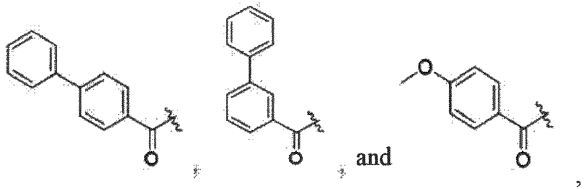 but is not limited thereto.

In yet another embodiment of the present invention, the chemotherapeutic agent and the compound may be included at a molarity ratio of 1:1 to 1:500, but is not limited thereto.

In yet another embodiment of the present invention, the cancer may be a cancer selected from the group consisting of lung cancer, breast cancer, blood cancer, colorectal cancer, pancreatic cancer, and combinations thereof, but is not limited thereto.

Moreover, the present invention provides a kit for preventing or treating cancer, comprising (i) a compound represented by the following General Formula; and (ii) an anticancer drug as an active ingredient:

[General Formula] X-AQTGTGKT

(In the General Formula, A represents alanine, Q represents glutamine, T represents threonine, G represents glycine, K represents lysine, and X may be absent; or
at least one selected from the group consisting of

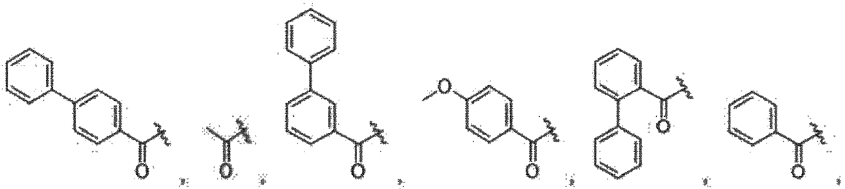

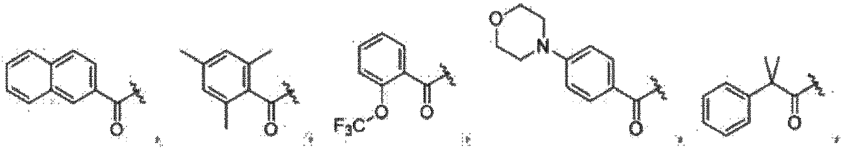

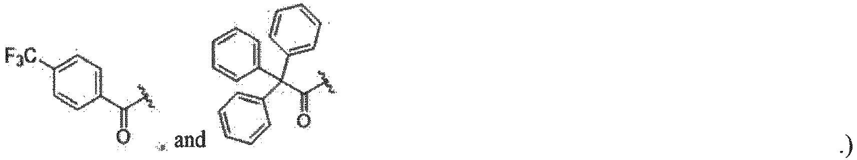

Moreover, the present invention provides a pharmaceutical composition for enhancing the anticancer effect of an anticancer drug, comprising a compound represented by the following General Formula as an active ingredient:

[General Formula] X-AQTGTGKT

(In the General Formula, A represents alanine, Q represents glutamine, T represents threonine, G represents glycine, K represents lysine, and X may be absent; or at least one selected from the group consisting of

In one embodiment of the present invention, the composition may be administered simultaneously, separately, or sequentially with the anticancer drug, but is not limited thereto.

In another embodiment of the present invention, the cancer may be a cancer selected from the group consisting of lung cancer, breast cancer, blood cancer, colorectal cancer, pancreatic cancer, and combinations thereof, but is not limited thereto.

Furthermore, the present invention provides a method for preventing or treating cancer, comprising the steps of administering (i) a compound represented by the General Formula; and (ii) a composition comprising an anticancer drug as an active ingredient, to a subject in need thereof. The compound and the anticancer drug may be administered in an effective dose, but are not limited thereto.

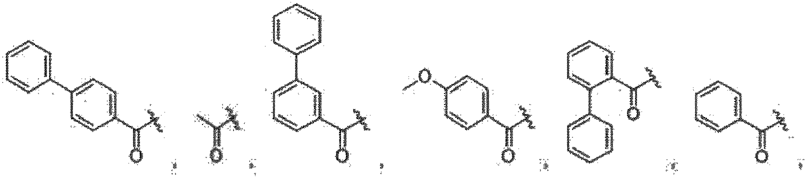

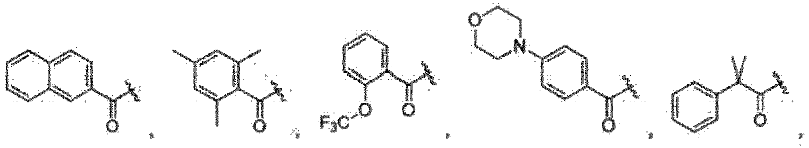

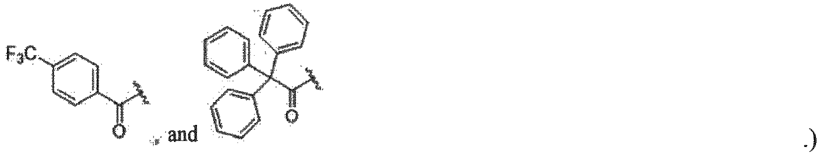

Moreover, the present invention provides a method for enhancing the anticancer effect of an anticancer drug, comprising the step of administering a compound represented by the General Formula to a subject in need thereof. The compound may be administered in an effective dose, but is not limited thereto.

Additionally, the present invention provides a use of a compound represented by the General Formula for manufacturing an enhancer of the anticancer effect of an anticancer drug.

Moreover, the present invention provides a use of a compound represented by the General Formula for enhancing the anticancer effect of an anticancer drug. The use for enhancing the anticancer effect of the anticancer drug comprises a use for inhibiting side effects of the anticancer drug.

Additionally, the present invention provides a use for manufacturing a drug for treating cancer, of a composition comprising (i) a compound represented by the General Formula; and (ii) an anticancer drug as an active ingredient.

Moreover, the present invention provides a use for preventing or treating cancer, of a composition comprising (i) a compound represented by the General Formula; and (ii) an anticancer drug as an active ingredient.

### [Advantageous Effects]

The present invention relates to a pharmaceutical composition for preventing or treating cancer and a composition for enhancing the anticancer effect of an anticancer drug, wherein the compositions comprise oligopeptide AQTGTGKT and analog compounds thereof as active ingredients based on the autophagy mechanism involved in the resistance of conventional anticancer drugs as a tumor promoter. The compounds according to the present invention may selectively bind to a protein target involved in the upper stage of autophagy only when cancer progresses, thereby over-activating autophagy, specifically targeting only cancer cells without affecting normal cells. Therefore, it is possible to maximize the inhibitory effect on cancer cell viability while minimizing the side effects of the effect of single administration of conventional anticancer drugs in patients resistant to anticancer drugs.

More specifically, when oligopeptide AQTGTGKT and its analog compounds are co-administered with conventional anticancer drugs, they demonstrate an enhanced anticancer effect and may significantly suppress cancer cell viability with excellent anticancer effects even with low concentrations of the drug. This may minimize side effects such as functional and active damage appearing in normal tissues, bone marrow dysfunction, gastrointestinal disturbance, alopecia, and anticancer drug resistance. In addition, oligopeptides have the advantage of being small in molecular weight compared to antibodies, reducing the concern of immune responses, and easy penetration into tissues. Therefore, it is anticipated that oligopeptides may be utilized in combination with anticancer drugs for various types of cancer.

### [Brief Description of the Drawings]

FIG. 1A shows the results of an analysis of the cell viability inhibitory effect resulting from the combined treatment of the compound 3-PhPh-AQTGTGKT according to the present invention and Osimertinib in the lung cancer cell line H1975, using an MTT assay (**p* < 0.05, ***p* < 0.01, ****p* < 0.001; the same hereafter).
FIG. 1B shows the results of an analysis of the cell viability inhibitory effect resulting from the combined treatment of the compound 4-PhPh-AQTGTGKT according to the present invention and Osimertinib in the lung cancer cell line H1975, using an MTT assay.
FIG. 1C represents the results of an analysis of the cell viability inhibitory effect resulting from the combined treatment of the compound Ac-AQTGTGKT according to the present invention and Osimertinib in the lung cancer cell line H1975, using an MTT assay.
FIG. 1D represents the results of an analysis of the cell viability inhibitory effect resulting from the combined treatment of the compound 4-MeOPh-AQTGTGKT according to the present invention and Osimertinib in the lung cancer cell line H1975, using an MTT assay.
FIG. 1E represents the results of an analysis of the cell viability inhibitory effect resulting from the combined treatment of the compound 4-PhPh-AQTGTGKT according to the present invention and Alimta in the lung cancer cell line H 1975, using an MTT assay.
FIG. 1F represents the results of an analysis of the cell viability inhibitory effect resulting from the combined treatment of the compound 3-PhPh-AQTGTGKT according to the present invention and Alimta in the lung cancer cell line H1975/OR, using an MTT assay.
FIG. 1G represents the results of an analysis of the cell viability inhibitory effect resulting from the combined treatment of the compound 3-PhPh-AQTGTGKT according to the present invention and Osimertinib in the lung cancer cell line H1975/OR, using an MTT assay.
FIG. 1H represents the results of an analysis of the cell viability inhibitory effect resulting from a triple combined treatment of the compound 3-PhPh-AQTGTGKT according to the present invention, Osimertinib, and Alimta in the lung cancer cell line H1975, using an MTT assay.
FIG. 2A represents the results of an analysis of the cell viability inhibitory effect resulting from a treatment of the compound AQTGTGKT according to the present invention and Osimertinib in the lung cancer cell line H820, using an MTT assay.
FIG. 2B represents the results of an analysis of the cell viability inhibitory effect resulting from the combined treatment of the compound 3-PhPh-AQTGTGKT according to the present invention and Osimertinib in the lung cancer cell line H820, using an MTT assay.
FIG. 3 represents the results of an analysis of the cell viability inhibitory effect resulting from the combined treatment of the compound 3-PhPh-AQTGTGKT according to the present invention and Osimertinib in the lung cancer cell line PC9/OR, using an MTT assay.
FIG. 4A represents the results of an analysis of the cell viability inhibitory effect resulting from the combined treatment of the compound AQTGTGKT according to the present invention and Olaparib in the breast cancer cell line HCC1937, using an MTT assay.
FIG. 4B represents the results of an analysis of the cell viability inhibitory effect resulting from the combined treatment of the compound 3-PhPh-AQTGTGKT according to the present invention and Olaparib in the breast cancer cell line HCC1937, using an MTT assay.
FIG. 4C represents the results of an analysis of the cell viability inhibitory effect resulting from the combined treatment of the compound 4-MeOph-AQTGTGKT according to the present invention and Cisplatin in the breast cancer cell line HCC1937, using an MTT assay.
FIG. 4D represents the results of an analysis of the cell viability inhibitory effect resulting from the combined treatment of the compound 3-PhPh-AQTGTGKT according to the present invention and Cisplatin in the breast cancer cell line HCC1937, using an MTT assay.
FIG. 5A represents the results of a comparative analysis of the cell viability inhibitory effect depending on the timing of the combined treatment for exploring the optimal combined regimen of the compound 3-PhPh-AQTGTGKT according to the present invention and Osimertinib in cancer cells.
FIG. 5B represents the results of a comparative analysis of the cell viability inhibitory effect depending on the duration of the combined treatment for specific exploration of the optimal combined regimen of the compound 3-PhPh-AQTGTGKT according to the present invention and Osimertinib in the lung cancer cell line PC9/OR.
FIG. 6A represents the results of a comparative analysis of the tumor viability inhibitory effect resulting from the combined treatment of the compound 3-PhPh-AQTGTGKT according to the present invention and Osimertinib after forming a tumor by inoculating the lung cancer cell line H820 into a nude mouse.
FIG. 6B represents the results of a comparative analysis of the tumor viability inhibitory effect resulting from the combined treatment of the compound 3-PhPh-AQTGTGKT according to the present invention and Osimertinib after forming a tumor by inoculating the lung cancer cell line H1975 into a nude mouse.
FIG. 7 represents the results of a comparative analysis of the inhibitory effect on p-Beclin1S15 in the tumor after performing the combined treatment of the compound 3-PhPh-AQTGTGKT according to the present invention and Osimertinib, and then removing the tumor tissue from the tumor formed by inoculating the lung cancer cell line H820 into a nude mouse.

### [Modes of the Invention]

The inventors have completed the invention by confirming that the oligopeptide AQTGTGKT and its amidated analog exhibit enhanced anticancer effects when used in combination with conventional anticancer drugs in various cancer cell lines.

In one embodiment of the present invention, it was confirmed that when a targeted anticancer drug and/or a chemotherapeutic agent are used in combination with a compound according to the present invention in lung cancer cell lines, the anticancer effect significantly increases compared to when each is administered alone (refer to Example 1).

In another embodiment of the present invention, it was confirmed that when a targeted anticancer drug or a chemotherapeutic agent is used in combination with the compound according to the present invention in breast cancer cell lines, the anticancer effect significantly increases compared to when each is administered alone (refer to Example 2).

In yet another embodiment of the present invention, as a result of analyzing the tumor viability inhibitory effect according to the combined treatment of Osimertinib and 3-PhPh-AQTGTGKT, it was found that the anticancer effect was further enhanced when Osimertinib and 3-PhPh-AQTGTGKT were combined and then Osimertinib was used alone, compared to when Osimertinib was administered alone and then 3-PhPh-AQTGTGKT was added for co-administration (refer to Example 3.1).

In yet another embodiment of the present invention, as a result of analyzing the tumor viability inhibitory effect according to the combined treatment of Osimertinib and 3-PhPh-AQTGTGKT, it was found that the anticancer effect was further enhanced when Osimertinib and 3-PhPh-AQTGTGKT were continuously combined, compared to when Osimertinib and 3-PhPh-AQTGTGKT were combined and then Osimertinib was administered alone (refer to Example 3.2).

In yet another embodiment of the present invention, using a heterotransplant lung cancer animal model produced with H820 or H1975 lung cancer cell lines, the effect of co-administration of Osimertinib and 3-PhPh-AQTGTGKT was examined, and it was confirmed that co-administration of the compound and the anticancer drug exhibited a significantly increased tumor viability inhibitory effect compared to when each was administered alone (refer to Example 4).

In yet another embodiment of the present invention, as a result of analyzing the autophagy action according to the co-administration of Osimertinib and 3-PhPh-AQTGTGKT, it was confirmed that the expression of p-Beclin1S15 was markedly decreased in the group co-administered with Osimertinib and 3-PhPh-AQTGTGKT compared to when each was administered alone (refer to Example 5).

Thus, the present invention provides (i) a compound represented by the following General Formula; and (ii) a pharmaceutical composition for preventing or treating cancer, comprising an anticancer drug as an active ingredient:

[General Formula] X-AQTGTGKT

(In the General Formula, A represents alanine, Q represents glutamine, T represents threonine, G represents glycine, K represents lysine, and

X is absent; or at least one selected from the group consisting of

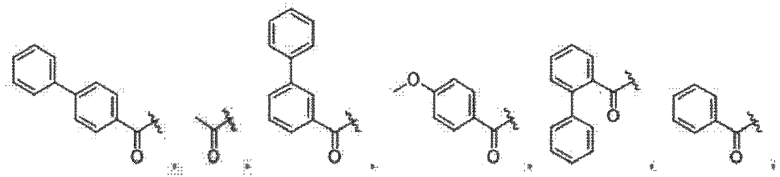

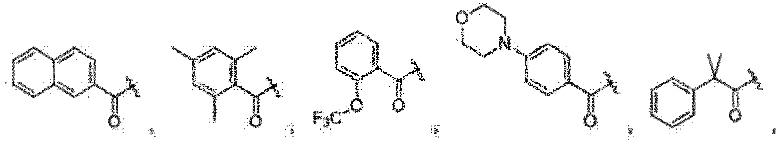

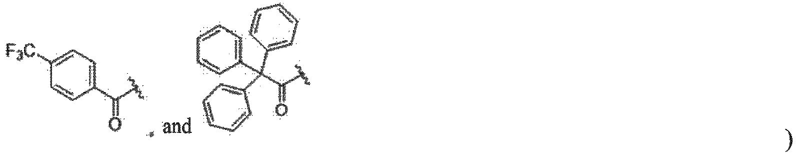

Preferably, the X may be absent; or at least one selected from the group consisting of 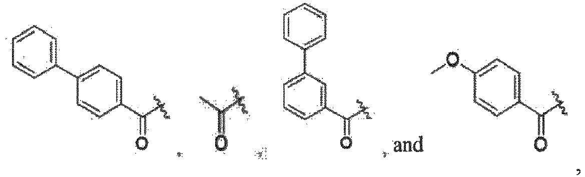 but is not limited thereto.

Additionally, the present invention provides a pharmaceutical composition for enhancing the anticancer effect of an anticancer drug, comprising a compound represented by the General Formula as an active ingredient.

Moreover, the present invention provides a pharmaceutical composition for suppressing side effects of an anticancer drug, comprising the compound represented by the General Formula as an active ingredient. The suppression of side effects of the anticancer drug comprises inhibition of drug resistance of the anticancer drug.

Also, the present invention provides a pharmaceutical composition for co-administration of an anticancer drug, comprising the compound represented by the General Formula as an active ingredient.

The absence of X in the General Formula means that the compound represented by the General Formula is AQTGTGKT. In the present invention, the X may be 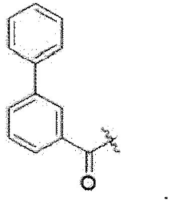 The compound where the X is 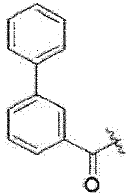 may be represented as "3-PhPh-AQTGTGKT" in this specification. Other names of the compound according to the present invention are listed in Table 1.

The term "oligopeptide" as used herein refers to a linear molecule formed by amino acid residues linked together by peptide bonds. The amidated oligopeptides of the present invention may be produced by chemical synthesis methods known in the art, in addition to molecular and biological methods (for example, solid-phase synthesis techniques (Merrifield, J. Amer. Chem. Soc. 85: 2149-54(1963); Stewart, et al., Solid Phase Peptide Synthesis, 2nd. ed., Pierce Chem. Co.: Rockford, 111(1984))).

The scope of the compound according to the present invention may comprise its pharmaceutically acceptable salts. The term "pharmaceutically acceptable" as used herein means compounds that are suitable for use in contact with the tissue of a subject (e.g., a human), where the benefit/risk ratio is reasonable without excessive toxicity, irritation, allergic reactions, or other problems or complications, and within the range of sound medical judgment. The pharmaceutically acceptable salts may comprise, for example, acid addition salts formed by a pharmaceutically acceptable free acid, and pharmaceutically acceptable metal salts.

Examples of suitable acids comprise hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzene sulfonic acid, etc. Acid addition salts may be manufactured by conventional methods, for example, by dissolving the compound in an excess of an acid aqueous solution and precipitating this salt using a water-miscible organic solvent such as methanol, ethanol, acetone, or acetonitrile. Acid addition salts may also be prepared by heating an equimolar amount of the compound and an acid in water or alcohol and subsequently evaporating the mixture to dryness, or by aspiration-filtration of the precipitated salt.

Salts derived from suitable bases may comprise alkali metals such as sodium, potassium, alkaline earth metals such as magnesium, and ammonium, but are not limited thereto. Alkali metal or alkaline earth metal salts, for example, may be obtained by dissolving the compound in an excess of alkali metal hydroxide or alkaline earth metal hydroxide solution, filtering off the insoluble compound salt, and then evaporating and drying the filtrate. Here, it is pharmaceutically suitable to manufacture particularly sodium, potassium, or calcium salts as the metal salts, and the corresponding silver salts may be obtained by reacting an alkali metal or alkaline earth metal salt with a suitable silver salt (for example, silver nitrate).

The scope of the compound of the present invention may comprise not only pharmaceutically acceptable salts, but also all isomers, hydrates, and solvates that may be produced by conventional methods.

The compound may have non-aromatic double bonds and at least one asymmetric center. Therefore, the compound may occur as racemates and racemate mixtures, single enantiomers, individual diastereomers, diastereomer mixtures, and cis- or trans-isomers. All these forms of isomers are considered.

Furthermore, the scope of the compound according to present invention may comprise biologically equivalent substances having variations in the amino acid sequence that exert equivalent biological activity to the compound of the present invention. Such variations in the amino acid sequence may be based on the relative similarity of amino acid side chain substitutes, such as hydrophobicity, hydrophilicity, charge, and size. By analyzing the size, shape, and type of amino acid side chain substitutes, it may be known that alanine and glycine have similar sizes; lysine has a positively charged residue; and glutamine and threonine are uncharged. Therefore, based on these considerations, alanine and glycine; and glutamine and threonine are biologically equivalent substances.

In introducing variations, the hydropathic index of amino acids may be considered. Each amino acid has been assigned a hydropathic index based on hydrophobicity and charge as follows: isoleucine (+4.5); valine (+4.2); leucine (+3.8); phenylalanine (+2.8); cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

The hydropathic index of amino acid is very important in conferring interactive biological functions of proteins. It is a recognized fact that a substitution with an amino acid with a similar hydropathic index may maintain similar biological activity. When introducing variations by referring to the hydropathic index, substitutions are made between amino acids showing a difference in the hydropathic index, preferably within ±2, more preferably within ±1, and even more preferably within ±0.5.

Meanwhile, it is also well known that substitutions between amino acids with similar hydrophilicity values result in proteins with biologically equivalent activity. As disclosed in U.S. Patent No. 4,554,101, the following hydrophilicity values have been assigned to each amino acid residue: arginine (+3.0); lysine (+3.0); aspartic acid (+3.0±1); glutamic acid (+3.0±1); serine (+0.3); asparagine (+0.2); glutamine (+0.2); glycine (0); threonine (-0.4); proline (-0.5+1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4).

In the case of introducing mutations by referring to the hydrophilicity values, desirably, substitutions are made between amino acids showing a difference in hydrophilicity values within ±2, more desirably within ±1, and even more desirably within ±0.5.

Amino acid exchanges in a protein that do not globally change the activity of the molecule are known in the field (H. Neurath, R.L.Hill, The Proteins, Academic Press, New York, 1979). The most common exchanges occur between the amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, AlalGlu, and Asp/Gly.

Considering mutations with the aforementioned biologically equivalent activity, the protein of the amino acid sequence (AQTGTGKT) of the compound represented by the General Formula of the present invention is interpreted to also comprise sequences showing substantial identity. Substantial identity herein means a sequence showing a minimum of 62.5% homology, more desirably at least 75% homology, and most desirably at least 87.5% homology when aligning the sequence of the present invention with any other sequence as much as possible and analyzing the aligned sequences using an algorithm commonly used in the industry. Alignment methods for sequence comparison are known in the industry.

The term "co-administration" as used herein may be achieved by administering the individual components of the therapeutic regimen simultaneously, sequentially, or individually. Co-administration may be obtained by methods such as administering two or more drugs simultaneously or sequentially, or alternatively at fixed or undefined intervals, and although not limited thereto, co-administration therapy may be defined as providing a superior therapeutic effect that may be measured through response degree, response speed, disease progression period or survival period, which is better than the effect that may be obtained by administering one or more of the components of the co-administration therapy at a conventional dose.

The term "anticancer drug" in this specification collectively refers to substances used for the treatment of malignant tumors. Most anticancer drugs intervene in various metabolic pathways of cancer cells, mainly inhibiting nucleic acid synthesis or exhibiting anticancer activity. Currently anticancer drugs used for cancer treatment are classified into six categories depending on their biochemical mechanisms of action, namely alkylating agents, antimetabolites, antibiotics, vinca alkaloids, hormonal drugs, and others, but the anticancer drug according to the present invention may not be included in these categories.

In the present invention, the anticancer drug may be at least one selected from the group consisting of targeted anticancer drugs and chemotherapeutic agents, but is not limited thereto.

The present invention may be co-administered with a targeted anticancer drug. In the present invention, the "targeted anticancer drug" refers to a preparation that exhibits an anticancer effect by interfering with molecular activities involved in the growth and occurrence of cancer, targeting proteins or genes specifically altered in cancer cells or cancer tissues. The targeted anticancer drug may be at least one selected from the group consisting of tyrosine kinase inhibitor (TKI), poly-ADP ribose polymerase inhibitor (PARP inhibitor), angiogenesis inhibitor, cyclin-dependent kinases 4/6 inhibitor (CDK4/6 inhibitor), hormonal therapy drug, and antibody-drug conjugate, but is not limited thereto. Preferably, the targeted anticancer drug according to the present invention may be a monoclonal antibody anticancer drug, but is not limited thereto.

In particular, the tyrosine kinase inhibitor may be a targeted drug for at least one selected from the group consisting of epidermal growth factor receptor (EGFR), anaplastic lymphoma kinase (ALK), ROS Proto-Oncogene 1 (ROS1), B-Raf Proto-Oncogene (BRAF), human epidermal growth factor receptor 2 (HER2), Ret Proto-Oncogene (RET), Neurotrophic Receptor Tyrosine Kinase 1 (NTRK1), Mesenchymal-Epithelial Transition factor (MET), and Neuregulin 1 (NRG1), and non-limiting examples thereof comprise Osimertinib, Afatinib, Brigatinib, Dasatinib, Dacomitinib, Erlotinib, Gefitinib, Lapatinib, Neratinib, Vandetanib, Icotinib, Varitinib, Tesevatinib, Canertinib, Naquotinib, Pelitinib, Poziotinib, Rociletinib, Nazartinib, Allitinib; ALS-1306, Pyrotinib, Tyrphostin, Crizotinib, Ceritinib, Entrectinib, Dabrafenib, Trametinib, Alectinib, Lorlatinib, Larotectinib, Lasertinib, Olmutinib, AG 1478, CUDC-101, MTKi-327 (JNJ-26483327), CL-387785 (EKI-785), CNX-2006, PD168393, TAK285, WZ4002, and AV-412 (MP-412).

In addition, the PARP inhibitor may be at least one selected from the group consisting of Olaparib, Rucaparib, Talazoparib, Veliparib, and Niraparib, but is not limited thereto.

Also, the CDK4/6 inhibitor may be at least one kind selected from the group consisting of Trilaciclib, Palbociclib, Ribociclib, and Abemaciclib, but is not limited thereto.

In addition, the hormonal therapy drug may be at least one selected from the group consisting of Tamoxifen, Toremifene, Fulvestrant, Goserelin, Leuprolide, Anastrozole, Letrozole, and Exemestane, but is not limited thereto.

Furthermore, the targeted anticancer drug according to the present invention may be antibody-drug conjugates (ADCs). The ADC comprises a conjugation of an antibody that binds to a specific target antigen on the surface of cancer cells, and a drug having a potent cytotoxic effect, utilizing the selectivity of the antibody for the target and the potent cytotoxic activity of the drug, which allows the drug to selectively act only on cancer cells, thereby enhancing the therapeutic effect and reducing side effects.

Preferably, the ADC may be selected from Sacituzumab govitecan and Ladiratuzumab, but is not limited thereto.

In addition, the targeted anticancer drug according to the present invention may be selected from Daratumumab, Trastuzumab, and Rituximab, but is not limited thereto.

In addition, the present invention may be administered with a chemotherapeutic agent. The term "chemotherapeutic agent" as used herein refers to a first-generation anticancer drug, also called a "cytotoxic anticancer drug" or "chemotherapeutic anticancer drug". Non-limiting examples of the chemotherapeutic agent comprise Alimta, Oxaliplatin, Pemetrexed, Cisplatin, Gemcitabine, Carboplatin, Fluorouracil (5-FU), Cyclophosphamide, Paclitaxel, Vincristine, Etoposide, Doxorubicin, etc.

Meanwhile, the compound according to the present invention may enhance the anticancer effect of the anticancer drug and reduce side effects. This is because an appropriate co-administration may minimize the dose of the anticancer drug with side effects. Here, "enhancing the anticancer effect" means all effects that may enhance the function of the anticancer drug, such as inhibiting tumor viability, inhibiting tumor metastasis, inhibiting tumor recurrence, and even inhibiting the resistance or tolerance of cancer cells to the anticancer drug, thereby enhancing the anticancer effect. That is, the compound according to the present invention may be used as a compound for co-administration with the disclosed anticancer drug for the purpose of enhancing the anticancer effect. Thus, the compound according to the present invention may be used for the purpose of co-administration with the anticancer drug and may enhance the anticancer effect of the anticancer drug.

In the present invention, the compound or a composition comprising the same may be administered simultaneously, separately, or sequentially with an anticancer drug. Even in the case where it is administered sequentially with the anticancer drug, the order of administration is not limited, but the administration method may be appropriately adjusted depending on the type of cancer, the type of anticancer drug, the patient's condition, etc.

In one embodiment of the present invention, when the compound and Osimertinib were simultaneously administered in cancer cells initially, the inhibitory effect on the viability of cancer cells was highest. Therefore, the compound may be administered before or simultaneously with the administration of the anticancer drug. Also, when the compound of the present invention is continuously co-administered with the anticancer drug, the anticancer effect was better compared to when the anticancer drug was treated alone after co-administration. Thus, if the purpose is to maximize the anticancer effect, it is preferable to continuously co-administer the compound of the present invention with the anticancer drug.

The amount of the compound or the anticancer drug in the composition of the present invention may be appropriately adjusted according to the symptoms of the disease, the progress of the symptoms, the patient's condition, etc., for example, 0.0001 to 99.9 weight%, or 0.001 to 50 weight% based on the total weight of the composition, but it is not limited thereto. The ratio is based on the dry weight excluding the solvent.

In the present invention, the anticancer drug may be included at a concentration of 0.1 to 10 µM, 0.1 to 9 µM, 0.1 to 8 µM, 0.1 to 7 µM, 0.1 to 6 µM, 0.1 to 5 µM, 0.1 to 4 µM, 0.1 to 3 µM, 0.1 to 2 µM, or 0.1 to 1 µM, compared to the total composition, but it is not limited thereto.

Also, in the present invention, the compound represented by the General Formula may be included at a concentration of 1 to 50 µM, 1 to 40 µM, 1 to 30 µM, 1 to 20 µM, 1 to 15 µM, 1 to 12 µM, 1 to 10 µM, 1 to 9 µM, 1 to 8 µM, 1 to 7 µM, 1 to 6 µM, 1 to 5 µM, 1 to 4 µM, 1 to 3 µM, 1 to 2 µM, 2 to 12 µM, or 2 to 11 µM compared to the total composition, but it is not limited thereto.

In addition, the composition according to the present invention may be a mixture form in which the compound and the anticancer drug are mixed, for simultaneous administration of the compound and the anticancer drug.

Moreover, the composition according to the present invention may be in a form where the compound and the anticancer drug are each formulated to be administered simultaneously or sequentially. In this case, the composition may be a pharmaceutical composition for simultaneous or sequential co-administration, comprising a first pharmaceutical composition comprising a pharmaceutically effective amount of the compound as an active ingredient; and a second pharmaceutical composition comprising a pharmaceutically effective amount of the anticancer drug as an active ingredient. Here, the order of administration for sequential administration is not restricted and may be appropriately adjusted according to the patient's condition.

That is, if the pharmaceutical composition is a pharmaceutical composition for sequential co-administration, the composition may be administered with the compound ("first component") first and then the anticancer drug ("second component"), or in the opposite order.

In one embodiment of the present invention, the anticancer drug may be a targeted anticancer drug, and X in the compound represented by the General Formula may be absent; or at least one selected from the group consisting of 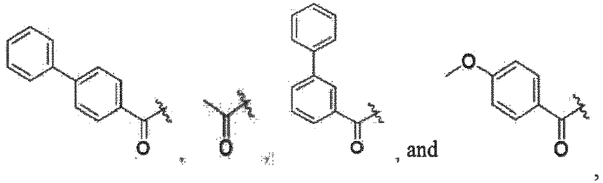 but is not limited thereto.

Preferably, the anticancer drug is a tyrosine kinase inhibitor, more preferably, Osimertinib, and X in the compound represented by the General Formula may be absent; or
at least one selected from the group consisting of 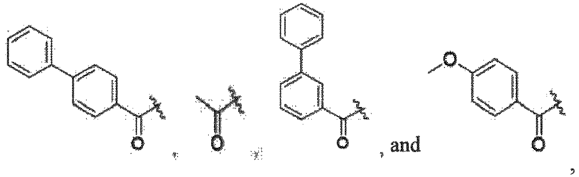 but is not limited thereto.

Preferably, the anticancer drug is a PARP inhibitor, more preferably Olaparib, and X in the compound represented by the General Formula may be absent; or X may be 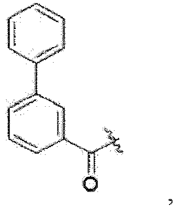 but is not limited thereto.

Also, the anticancer drug may be a chemotherapeutic agent, and the compound represented by the General Formula may be one or more selected from the group consisting of 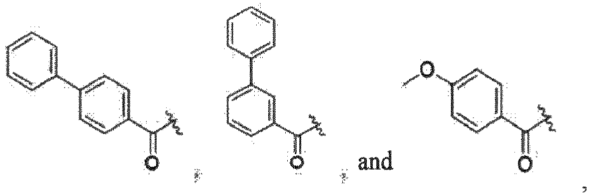 but is not limited thereto.

Preferably, the anticancer drug is Cisplatin, and the compound represented by the General Formula may have X as at least one selected from the group consisting of 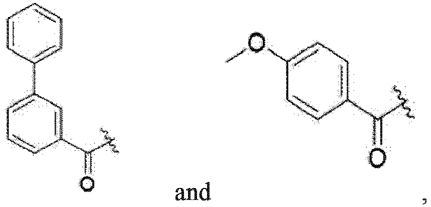 but is not limited thereto.

Preferably, the anticancer drug is Alimta, and the compound represented by the General Formula may have X as at least one selected from the group consisting of 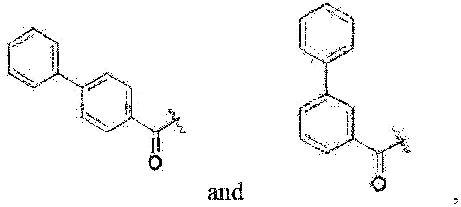 but is not limited thereto.

The anticancer drug and the compound may be included in a molarity ratio of 1:1 to 500, 1:1 to 400, 1:1 to 300, 1:1 to 200, 1:1 to 180, 1:1 to 150, 1:1 to 130, 1:1 to 120, 1:1 to 110, 1:1 to 100, 1:1 to 90, 1:1 to 80, 1:1 to 70, 1:1 to 60, 1:1 to 50, 1:1 to 40, 1:1 to 30, 1:1 to 20, 1:1 to 10, 1:1 to 9, 1:1 to 8, 1:1 to 7, 1:1 to 6, 1:1 to 5, 1:1 to 4, 1:1 to 3, or 1:1 to 2.

The compound according to the present invention may be used for preventing and/or treating cancer. The term "cancer" as used herein refers to uncontrolled cell growth, which results in a cluster of cells known as a tumor that infiltrates into surrounding tissues, and in severe cases, metastasizes to other organs of the body. Academically, it is also referred to as a neoplasm. Despite treatments with surgery, radiation, and chemotherapy, cancer is refractory chronic disease and often cannot be completely cured and causes suffering to patients, ultimately leading to death. There are various causes of cancer, which are divided into internal and external factors. Although it is not precisely clarified how a normal cell transforms into a cancer cell through some mechanism, it is known that a considerable number of cancers occur under the influence of external factors such as environmental factors. Internal factors include genetic factors and immunological factors, while external factors include chemical substances, radiation, and viruses. Genes related to cancer development include oncogenes and tumor suppressor genes, and cancer occurs when the balance between them is broken by the aforementioned internal or external factors.

The cancer may be a solid cancer or a blood cancer, and non-limiting examples thereof may be at least one selected from the group consisting of squamous cell carcinoma, lung cancer, lung adenocarcinoma, peritoneal cancer, skin cancer, skin or intraocular melanoma, rectal cancer, perianal cancer, esophageal cancer, small intestine cancer, endocrine gland cancer, parathyroid cancer, adrenal cancer, connective tissue sarcoma, ureteral cancer, blood cancer, liver cancer, stomach cancer, pancreatic cancer, glioma, cervical cancer, ovarian cancer, bladder cancer, hepatic tumor, breast cancer, colon cancer, colorectal cancer, endometrial or uterine cancer, salivary gland cancer, kidney cancer, prostate cancer, vaginal cancer, thyroid cancer, head and neck cancer, brain cancer, and more specifically, it may be cancer selected from the group consisting of lung cancer, breast cancer, blood cancer, colorectal cancer, pancreatic cancer, and combinations thereof.

In the present invention, the lung cancer may be a non-small cell lung carcinoma or a lung papillary adenocarcinoma.

Moreover, the breast cancer may be a hormone receptor (HR) positive breast cancer, but is not limited thereto. Additionally, the breast cancer may be a triple negative breast cancer.

Furthermore, the blood cancer may be leukemia, lymphoma, multiple myeloma, etc.

In yet another aspect of the present invention, the invention may provide a kit for enhancing the anticancer effect of an anticancer drug, comprising a compound represented by the General Formula.

Also, in another aspect of the present invention, the invention may provide a kit for preventing or treating cancer, comprising (i) a compound represented by the General Formula; and (ii) an anticancer drug as an active ingredient.

The kit according to the present invention may comprise, without limitation, other constituents, compositions, solutions, devices, etc., typically needed for preventing or treating cancer, in addition to the compound or anticancer drug, and specifically may comprise instructions for suitable use and storage of the compound according to the present invention.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "improvement" as used herein means all actions that reduce the degree of parameters related to a target disease, e.g., symptoms via administration of the composition according to the present invention.

The preventing and/or treating effect on the cancer not only comprises an effect of inhibiting the growth of cancer cells, but also comprises an effect of inhibiting the worsening of cancer caused by migration, invasion, metastasis, and the like.

The term "subject" as used herein refers to an object that requires prevention or treatment of a disease. For example, the subject may be a mammal, including humans, or non-human primates, mice, dogs, cats, horses, sheep, and cows.

Meanwhile, the pharmaceutical composition according to the present invention may further comprise a suitable carrier, excipient, and diluent which are commonly used in the preparation of pharmaceutical compositions. Also, it may be formulated into topical preparations, suppositories, sterile injection solutions, oral formulations such as tablets, granules, pills, capsules, suspensions, emulsions, syrups, and aerosols, using conventional methods.

As the carrier, the excipient, and the diluent that may be comprised in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used. For formulation, commonly used diluents or excipients such as fillers, thickeners, binders, wetting agents, disintegrants, and surfactants are used.

The term "administration" as used herein refers to providing a subject with a predetermined composition of the present invention by using an arbitrary appropriate method.

The pharmaceutical composition according to the present invention is administered in a pharmaceutically effective amount. In the present invention, "pharmaceutically effective amount" refers to an amount sufficient to treat diseases at a reasonable benefit/risk ratio applicable to medical treatment, and the effective dosage level may be determined according to factors comprising the type of patient's disease, severity, drug activity, sensitivity to the drug, administration time, administration route and excretion rate, treatment period, drugs used simultaneously, and other factors well known in the medical field. A desirable dosage may be chosen according to the subject's condition and weight, degree of disease, drug form, administration route and duration. As a specific example, the pharmaceutical composition may be administered divided into once or multiple times a day in the amounts of 0.001 to 1000 mg/kg, 0.01 to 100 mg/kg, 0.01 to 10 mg/kg, 0.1 to 10 mg/kg, or 0.1 to 1 mg/kg.

Considering all the aforementioned factors, it is important to administer an amount that may achieve maximum effect with a minimum amount without side effects, and this may be determined by a person skilled in the art. Specifically, the effective amount of the pharmaceutical composition according to the present invention may vary depending on the patient's age, sex, condition, weight, the absorbency, inactivity rate and excretion speed of the active ingredient in the body, type of disease, and drugs being administered concurrently.

The pharmaceutical composition of the present invention may be administered to a subject via various routes. All administration methods may be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like. The daily dose may be administered once or several times daily in divided doses.

Terms or words used in this specification and the scope of claims should not be interpreted exclusively in their conventional or dictionary meanings, and the principle that the inventor may appropriately define the concepts of the terms to best describe their own invention should guide interpretations, aligning the meanings and concepts with the technical thoughts of the present invention.

Hereinafter, preferable examples are presented to assist in understanding the present invention. However, the provided examples serve only to simplify the understanding of the present invention and the content of the present invention is not limited thereto.

### [Examples]

### Experimental Materials and Methods

### 1. Synthesis of AQTGTGKT Analog Compounds

Thirteen types of AQTGTGKT analog compounds according to the present invention were synthesized by commissioning to Sewonbiotech, and their chemical structures were confirmed by analyzing with ¹H NMR and UPLC-MS (ultraperformance liquid chromatography-mass spectrometry).

### 1.1. General Reaction

All reactions, unless otherwise mentioned, were carried out using commercially available materials and reagents without any additional reactions. The reactions were monitored by thin-layer chromatography (TLC) on silica gel plates (Keiselgel 60 F254, Merck) and/or ultra-high-performance liquid chromatography (UPLC). Visualization of spots on the TLC plates was achieved by UV light, staining the TLC plates in potassium permanganate and/or ninhydrin, and charring with a heat gun. All products were characterized using ¹H NMR and/or UPLC-MS.

### 1.2. Synthesis of Boc/OBn-TG

First, TG with a benzyl-protected functional group was synthesized according to Scheme 1 below. In the following schemes, compounds will be referred to as compound n according to the Arabic numeral (n) given below.

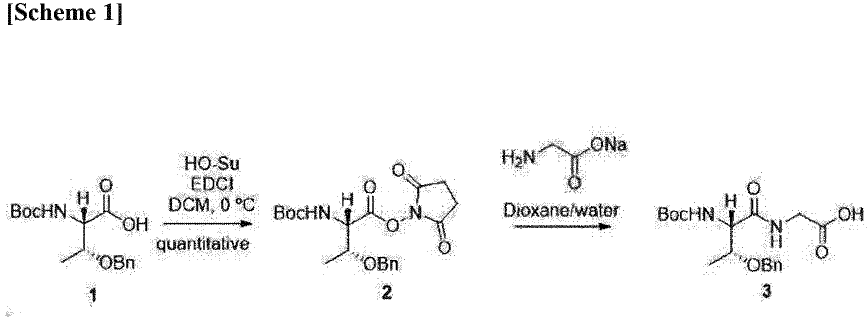

Specifically, BocThr(OBn)OH (compound 1; 25.0 g, 80.8 mmol, 1.0 equivalent) and NOSu (9.77 g, 84.8 mmol, 1.05 equivalent) were dissolved in dichloromethane (150 mL). The mixture was cooled to 0°C and kept under an inert atmosphere. Then, 1-(3-dimethylaminopropyl)-3-ethylcarbodiimide hydrochloride (16.3 g, 84.8 mmol, 1.05 equivalent) was added to the mixture. The mixture was warmed to room temperature and stirred for 20 hours. Subsequently, the mixture was washed with saturated aqueous NH₄Cl and the layers were separated. The organic layer was dried over MgSO₄ and concentrated under reduced pressure to give compound 2 as a pale yellow oil (35.7 g, >100% yield, assuming quantitative yield).

The compound 2, BocThr(OBn)OSu (32.8 g, 80.8 mmol, 1.0 equivalent), was dissolved in 1,4-dioxane (200 mL), and an aqueous solution of sodium glycinate hydrate in distilled water (100 mL) was added in one portion. After stirring at room temperature for 6 hours, the mixture was partitioned between ethyl acetate and saturated aqueous citric acid. The organic layer was dried over MgSO₄, filtered, and concentrated under reduced pressure. The residue was purified on a C18 (400 g) column with a gradient of 30-70% acetonitrile in water (0.1% formic acid). The desired fractions were combined and partitioned between ethyl acetate and saturated aqueous NaHCO₃. The organic layer was dried over MgSO₄, filtered, and concentrated under reduced pressure to give compound 3 as a pale yellow gum (21.9 g, 74% yield).

### 1.3. Synthesis of CBz/OBn/CO₂Bn-KT

KT with a benzyl-protected OH group was synthesized according to Scheme 2 below.

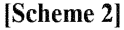

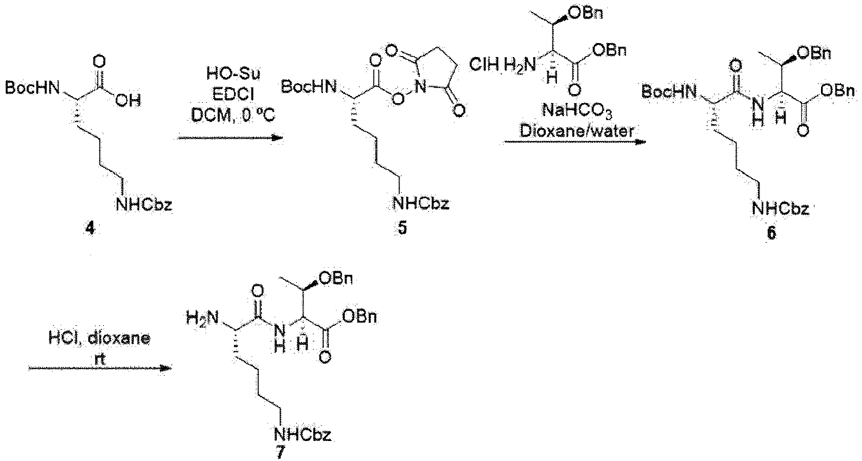

Specifically, BocLys(CBz)OH (compound 4; 27.0 g, 70.9 mmol, 1.0 equivalent) and NOSu (9.80 g, 85.1 mmol, 1.2 equivalent) were dissolved in dichloromethane (128 mL). The mixture was cooled to 0°C and kept under an inert atmosphere. Then, 1-(3-dimethylaminopropyl)-3-ethylcarbodiimide hydrochloride (16.3 g, 85.1 mmol, 1.05 equivalent) was added to the above-mentioned mixture. The mixture was warmed to room temperature and stirred for 20 hours. Subsequently, the mixture was washed with saturated aqueous NH₄Cl and the layers were separated. The organic layer was dried over MgSO₄ and concentrated under reduced pressure to give compound 5 as a light yellow oil (36.7 g, >100% yield, assuming quantitative yield).

Then, the compound 5 (BocLys(CBz)OSu; 36.7 g, 70.9 mmol, 1.0 equivalent) and Thr(OBn)OBn.HCl (25.0 g, 74.4 mmol, 1.05 equivalent) were dissolved in 1,4-dioxane (477 mL) at room temperature. An aqueous solution of NaHCO₃ (6.85 g, 81.5 mmol, 1.15 equivalent) in distilled water (326 mL) was added to the above solution. The resulting mixture was stirred at room temperature for 20 hours. The reaction mixture was diluted with ethyl acetate, and washed with 10% citric acid (aqueous) and brine. The organic layer was dried over Na₂SO₄, filtered, and concentrated under reduced pressure to give compound 6 as a yellow oily solid (55.9 g, >100% yield, assuming quantitative yield).

Finally, the compound 6 (BocLys(CBz)Thr(OBn)OBn; 55.9 g, 70.9 mmol, 1.00 equivalent) was dissolved in 1,4-dioxane (360 mL) and 4N HCl in 1,4-dioxane (177 mL) was added. The mixture was stirred overnight at room temperature. Then, an aqueous solution of saturated NaHCO₃ was added until the pH value reached 8. The above solution was extracted with ethyl acetate, the resulting organic solution was dried over Na₂SO₄, filtered, and then concentrated under reduced pressure to give compound 7 as a yellow oily solid (38.7 g, 97% yield).

### 1.4. Synthesis of CBz/OBn/OBn/CO₂Bn-TGKT

TG and KT synthesized in 1.2. and 1.3. respectively, were combined according to Scheme 3 to synthesize TGKT.

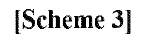

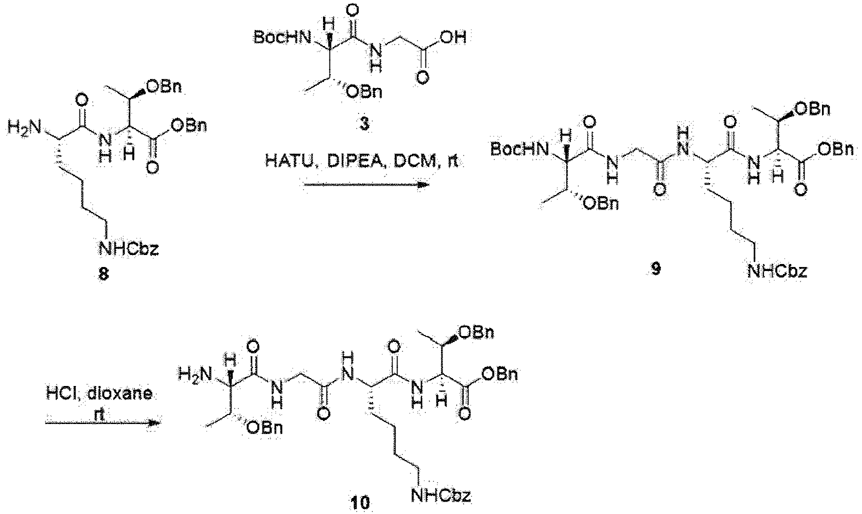

More specifically, *N,N*-Diisopropylethylamine (5.90 mL, 33.7 mmol, 2.2 equivalent) was added to a solution of BocThr(OBn)GlyOH (compound 3; 5.61 g, 15.3 mmol, 1.00 equivalent) and compound 8 (Lys(Cbz)Thr(OBn)OBn; 10.0 g, 15.3 mmol, 1.0 equivalent) in dichloromethane (50 mL). The mixture was stirred under an inert atmosphere at room temperature and HATU (7.00 g, 18.4 mmol, 1.20 equivalent) was added. The resulting mixture was stirred for 2 hours, then washed with saturated aqueous NH₄Cl, followed by washing with saturated aqueous NaHCO₃. The organic layer was dried over Na₂SO₄, filtered and then concentrated under reduced pressure to give compound 9 as a light orange oily solid (25.0 g, >100% yield, assuming quantitative yield).

The obtained BocThr(OBn)GlyLys(Cbz)Thr(OBn)OBn (compound 9; obtained from the previous step 13.9 g, 15.3 mmol, 1.0 equivalent) was dissolved in 1,4-dioxane (150 mL) under nitrogen at room temperature. 4N HCl in 1,4-dioxane (20 mL) was added to this solution. The resulting mixture was stirred at room temperature for 20 hours. The mixture was concentrated under reduced pressure and purified on a C18 (400 g) column using 20% acetonitrile (0.1% formic acid) in a water (0.1% formic acid) eluent. The desired fractions were combined and freeze-dried. The resulting powder was dissolved in saturated aqueous NaHCO₃ and dichloromethane and stirred for 15 minutes. The layers were separated, and the organic layer was dried over Na₂SO₄, filtered, and then concentrated under reduced pressure to give compound 10 as a colorless gum (10.9 g, 88% yield).

### 1.5. Synthesis of CBz/OBn/OBn/OBn/CO₂Bn-TGTGKT

TG (compound 3) and TGKT (compound 10) synthesized in 1.2. and 1.4. respectively, were combined according to Scheme 4 below to synthesize benzyl-protected TGTGKT.

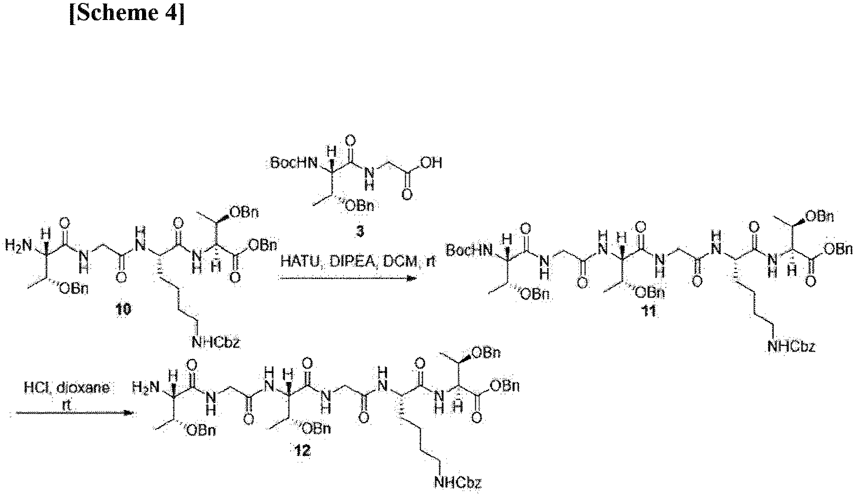

More specifically, *N,N*-Diisopropylethylamine (5.10 mL, 29.3 mmol, 2.2 equivalent) was added to a solution of BocThr(OBn)GlyOH (compound 3; 5_ 10 g, 14.0 mmol, 1.05 equivalent) and BocThr(OBn)GlyLys(Cbz)Thr(OBn)OBn (compound 10; 10.8 g, 13.3 mmol, 1.0 equivalent) in dichloromethane (100 mL). The mixture was stirred under an inert atmosphere at room temperature and HATU (5.60g, 14.7 mmol, 1.1 equivalent) was added. The resulting mixture was stirred for 2 hours, then washed with saturated aqueous NH₄Cl, followed by washing with saturated aqueous NaHCO₃. The organic layer was concentrated under reduced pressure to give compound 11 as a pale yellow gum (21.4 g, >100% yield, assuming quantitative yield).

Subsequently, the compound 11 (BocThr(OBn)GlyThr(OBn)GlyLys(Cbz)Thr(OBn)OBn; obtained from the previous step 15.4 g, 13.3 mmol, 1.00 equivalent) was dissolved in 1,4-dioxane (150 mL) under nitrogen at room temperature. 4N HCl in 1,4-dioxane (50 mL) was added to this solution, and the mixture was stirred at room temperature for 5 hours. The mixture was concentrated under reduced pressure and purified on a C18 (120 g) column using 20% acetonitrile (0.1% formic acid) in a water (0.1% formic acid) eluent. The desired fractions were combined, concentrated to half volume, and then partitioned between saturated aqueous NaHCO₃ and ethyl acetate. The layers were separated, and the organic layer was dried over Na₂SO₄, filtered, and then concentrated under reduced pressure to give compound 12 as a light gray solid (14.6 g, >100% yield, assuming quantitative yield).

### 1.6. Synthesis of CBz/OBn/OBn/OBn/CO₂Bn-QTGTGKT

The compound 12 (TGTGKT) synthesized in 1.5 above was further combined with Q according to Scheme 5 below to synthesize compound 14 (QTGTGKT).

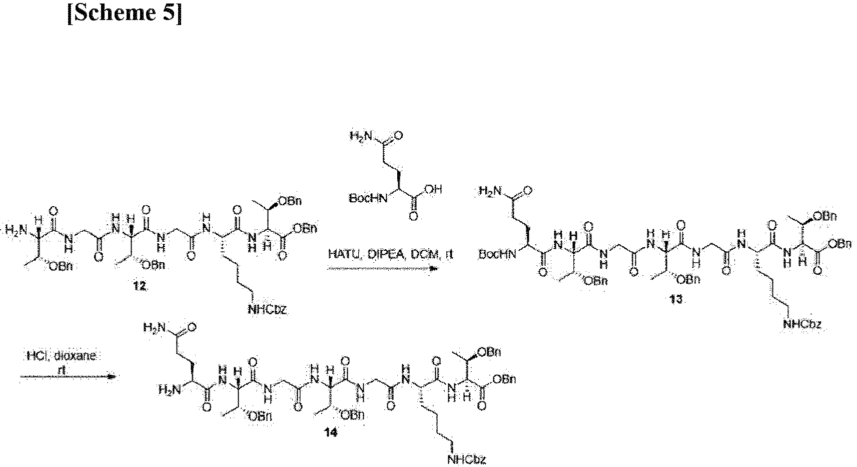

More specifically, to the solution of Thr(OBn)GlyThr(OBn)GlyLys(Cbz)Thr(OBn)OBn (compound 12; 12.7 g, 12.0 mmol, 1.0 equivalent) and BocGlnOH (3.25 g, 13.2 mmol, 1.1 equivalent) in ethyl acetate (150 mL) and N,N-Dimethylformamide (25 mL), *N,N*-Diisopropylethylamine (4.60 mL, 26.4 mmol, 2.2 equivalent) was added. The mixture was stirred under an inert atmosphere at room temperature, and HATU (5.47 g, 14.4 mmol, 1.20 equivalent) was added. The resulting mixture was stirred for 1 hour, then washed with saturated aqueous NH₄Cl. The organic layer was further extracted with dichloromethane. The organic layers were combined and concentrated under reduced pressure. The crude material was purified using a 400 g C18 column with a gradient of 20-100% acetonitrile (0.1% formic acid) in water (0.1% formic acid). The desired fractions were combined, then partitioned between ethyl acetate and a saturated aqueous solution of NaHCO₃. The organic layer was concentrated, and the residual water was removed by freeze-drying. A total of 12.9 g (89% total yield) of compound 13 was obtained from the combined fractions.

The compound 13 (BocGlnThr(OBn)GlyThr(OBn)GlyLys(Cbz)Thr(OBn) OBn; 7.00 g, 5.40 mmol, 1.00 equivalent) was dissolved in 1,4-dioxane (150 mL) under nitrogen at room temperature. To this solution, 4N HCl in 1,4-dioxane (43.5 mL) was added. The mixture was stirred at room temperature for 20 hours. The mixture was concentrated under reduced pressure and freeze-dried using a water/acetonitrile (2/1) solution. Finally, compound 14 was isolated as a pale yellow powder (6.36 g, 96% yield).

### 1.7. Synthesis of AQTGTGKT analogs

Except for 4-PhPh-AQTGTGKT, the remaining 6 analogs were synthesized by combining the final product of Scheme 5, compound 14, and the product of Scheme 6, compound 17n, according to Scheme 7 below.

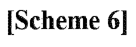

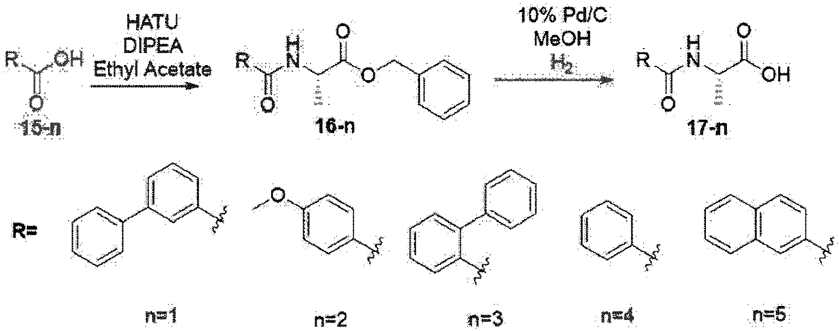

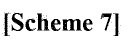

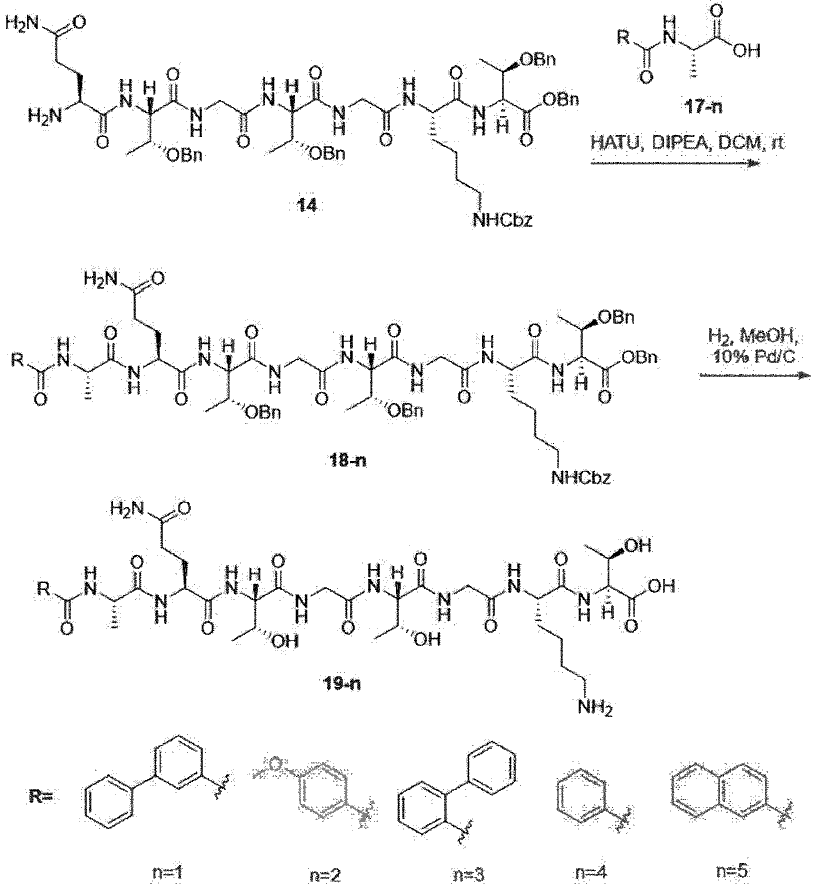

According to Scheme 7 above, finally, 3-PhPh-AQTGTGKT with a purity of over 90% (2.9 mg), 4-MeOPh-AQTGTGKT with a purity of 89% (7.0 mg), 2-PhPh-AQTGTGKT with a purity of over 95% (22.7 mg), Ph-AQTGTGKT with a purity of over 90% (23.2 mg), and Naphthyl-AQTGTGKT with a purity of 85% (23.2 mg) were obtained.

Herein, the synthesis process of each analog is described in detail.

### 1.7.1. Synthesis of 3-PhPh-AQTGTGKT

3-PhPh-AQTGTGKT (compound 19-1) was synthesized by reacting the compound 17-1, which was obtained according to Scheme 8 below, with the compound 14 according to Scheme 9 to ultimately produce the target compound 3-PhPh-AQTGTGKT.

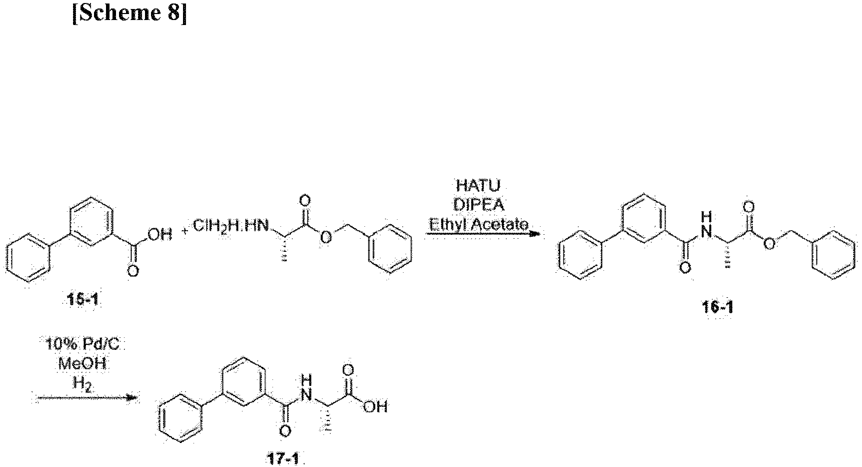

More specifically, H-Ala-OBzl.HCl (388 mg, 1.80 mmol, 1.2 equivalent) was suspended in ethyl acetate (10 mL), and N,N Diisopropylethylamine (653 µL, 3.75 mmol, 2.5 equivalent) was added. After stirring at room temperature for 5 minutes, HATU (855 mg, 2.25 mmol, 1.5 equivalent) and [1,1'-biphenyl]-3-carboxylic acid (297 mg, 1.50 mmol, 1 equivalent) were added and the mixture was stirred at room temperature for 2 hours. The reaction mixture was diluted with ethyl acetate and then washed with saturated aqueous NH₄Cl, saturated aqueous NaHCO₃, and saline. The obtained organic phase was dried (Na₂SO₄), filtered, and concentrated under reduced pressure. The residue was purified by a 2-40% ethyl acetate gradient in heptane on a 25 g column to give compound 16-1 as a colorless solid (493 mg, 91% yield).

Subsequently, a minimal amount of water-wet 10% Pd/C (49 mg) was added to a solution of compound 16-1 (3-PhPh-AlaOBn; 493 mg, 1.37 mmol) in methanol (30 mL). The mixture was stirred under a hydrogen atmosphere (balloon) for 2 hours. The mixture was filtered through a Celite pad, washed with methanol, and ethyl acetate. The obtained filtrate was concentrated under reduced pressure to give a colorless foam (337 mg, 91% yield) of compound 17-1, which was reacted with the compound 14 according to Scheme 9 below.

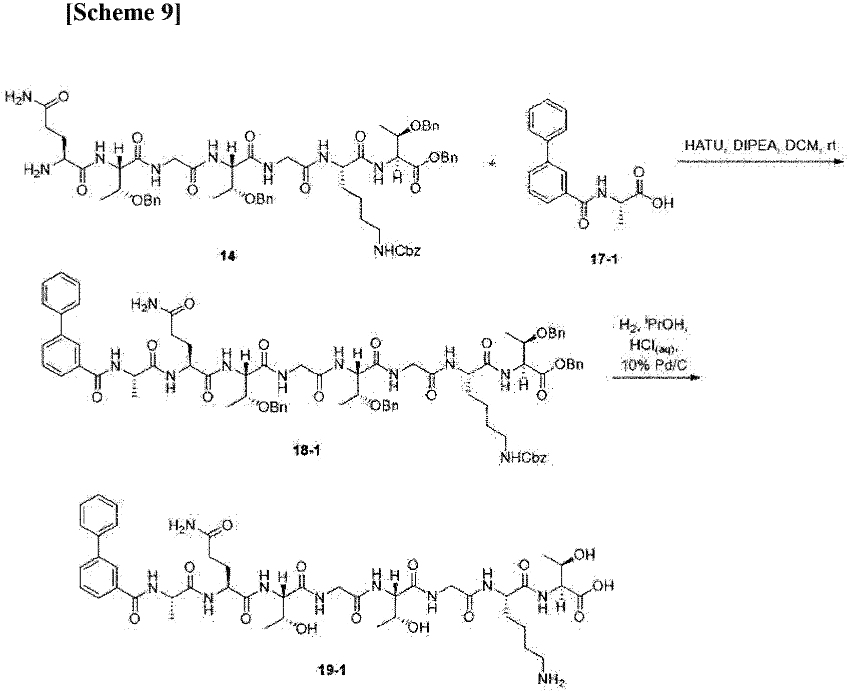

More specifically, HATU (106 mg, 0.278 mmol, 1.1 equivalent) was added to a suspension of *N,N-*Diisopropylethylamine (97.0 µL, 0.556 mmol, 2.2 equivalent) and GlnThr(OBn)GlyThr(OBn)GlyLys(Cbz) Thr(OBn)OBn (compound 14; 300 mg, 0.253 mmol, 1 equivalent) and 3-PhPh-AlaOH (compound 17-1; 68.0 mg, 0.253 mmol, 1 equivalent) in dichloromethane (20 mL). The mixture was stirred at room temperature for 2 hours, and the reaction mixture was washed with saturated aqueous NaHCO₃. The organic phase was concentrated under reduced pressure and the residue was purified on a 60g C18 column with a gradient of 40-100% acetonitrile (0.1% formic acid) in water, then freeze-dried to give compound 18-1 as a colorless solid (140 mg, 38% yield).

Subsequently, 10% Pd/C (85.0 mg) was added to a solution of 3-PhPh-AlaGlnThr(OBn)GlyThr(OBn)GlyLys(Cbz)Thr(OBn)OBn (compound 18-1; 85.0 mg, 59.1 µmol) in 2-propanol (10 mL) and 2M hydrochloric acid (aqueous, 0.5 mL). The mixture was stirred under a hydrogen atmosphere (balloon) for 4.5 hours and then the mixture was filtered through a 0.45 µm syringe filter. The obtained filtrate was concentrated under reduced pressure, and the residue was freeze-dried. The material was then purified on a 60g C18 column using a gradient of 5-50% acetonitrile (0.1% formic acid) in water, then freeze-dried to give target compound 19-1 as a colorless solid (2.9 mg, 5% yield).

The ¹H NMR data for compound 19-1 (3-PhPh-AQTGTGKT) was measured as follows:
¹H NMR (400 MHz; D2O): δ= 8.01-7.99 (m, 1H), 7.86-7.82 (m, 1H), 7.75-7.66 (m, 3H), 7.55 (t, J 7.7 Hz, 1H), 7.49 (t, J 7.5 Hz, 2H), 7.43-7.38 (m, 1H), 4.48-4.24 (m, 5H), 4.23-4.12 (m, 3H), 4.09 (d, J 3.8 Hz, 1H), 4.00- 3.96 (m, 2H), 3.88 (s, 2H), 2.90 (t, J 7.4Hz, 2H), 2.35 (t, J 7.5Hz, 2H), 2.15-2.06 (m, 1H), 2.03-1.91 (m , 1H), 1.84-1.72 (m, 1H), 1.70-1.52 (m, 3H), 1.45 (d, J 7.2Hz, 3H), 1.40-1.24 (m, 2H), 1.15-1.05 (m, 9H) , 16 exchangeable protons not visible.

### 1.7.2. Synthesis of 4-MeOPh-AQTGTGKT

The 4-MeOPh-AQTGTGKT (compound 19-2) was synthesized by reacting the compound 17-2, which was obtained according to Scheme 10 below, with the compound 14, according to Scheme 11, to produce the final target compound 4-MeOPh-AQTGTGKT.

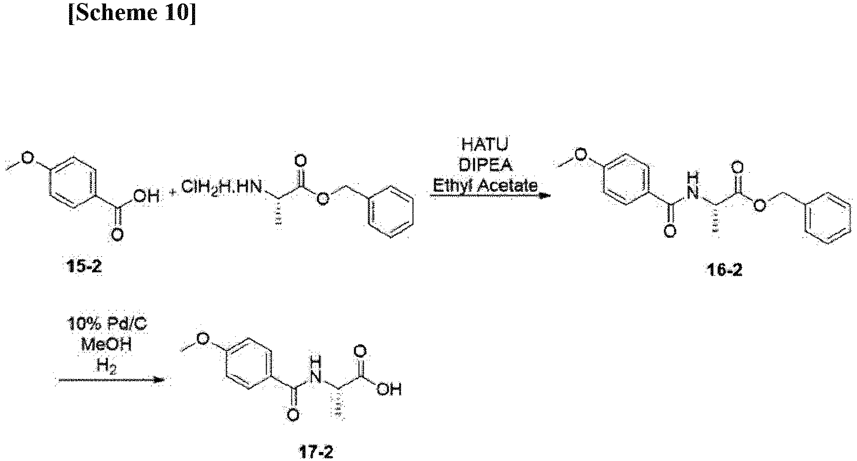

More specifically, H-Ala-OBzl.HCl (425 mg, 1.97 mmol, 1.2 equivalent) was suspended in ethyl acetate (10 mL) and *N,N*-Diisopropylethylamine (715 µL, 4.11 mmol, 2.5 equivalent) was added. After stirring at room temperature for 5 minutes, HATU (937mg, 2.46mmol, 1.5 equivalent) and 4-methoxybenzoic acid (250mg, 1.64mmol, 1 equivalent) were added and the mixture was stirred at room temperature for 2 hours. The reaction mixture was diluted with ethyl acetate, then washed with saturated aqueous NH₄Cl, saturated aqueous NaHCO₃, and brine. The organic phase was dried (Na₂SO₄), filtered and concentrated under reduced pressure. The residue was purified on a column of 25 g of silica gel with a gradient of 15-50% ethyl acetate in heptane to give compound 16-2 as a colorless solid (360 mg, 70% yield).

Then, a minimum amount of water-wet 10% Pd/C (18 mg) was added to a solution of 4-OMePh-AlaOBn (compound 16-2; 180 mg, 0.574 mmol) in methanol (15 mL). The mixture was stirred under a hydrogen atmosphere (balloon) for 110 hours. The mixture was then filtered through a Celite pad and washed with methanol. The obtained filtrate was concentrated under reduced pressure to give compound 17-2 as a colorless oil (128 mg, 100% yield), which was reacted with the compound 14 according to Scheme 11 below.

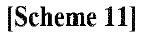

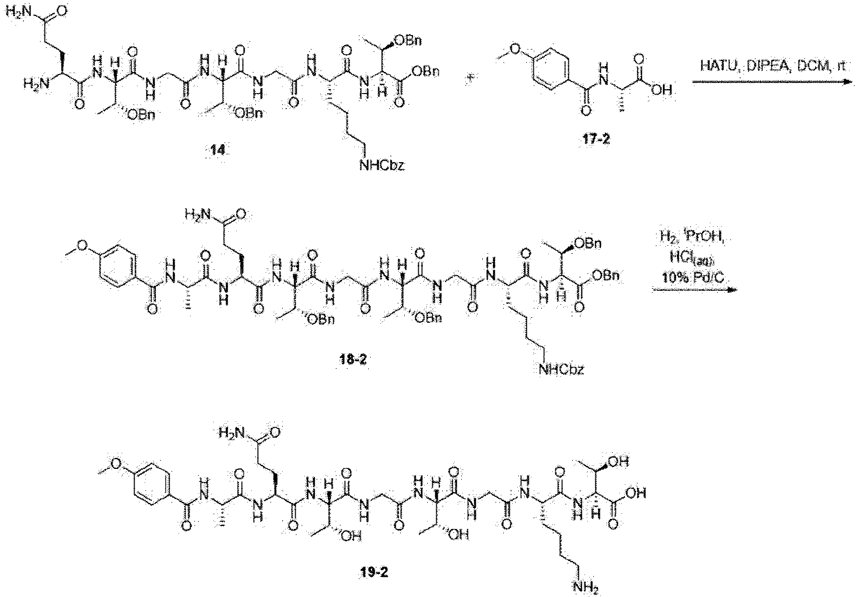

More specifically, *N*,*N*-Diisopropylethylamine (63.0 µL, 0.360 mmol, 2.2 equivalent) was added to a suspension of GlnThr(OBn)GlyThr(OBn)GlyLys (Cbz)Thr(OBn)OBn (compound 14; 200 mg, 0.164 mmol, 1 equivalent) and 4-OMePh-AlaOH (compound 17-2; 36.6 mg, 0.164 mmol, 1 equivalent) in dichloromethane (20 mL) with HATU (74.5 mg, 0.196 mmol, 1.2 equivalent). The mixture was stirred at room temperature for 64 hours, and the reaction mixture was diluted with methanol, then washed with saturated aqueous NH₄Cl, saturated aqueous NaHCO₃, and water. The organic matter was concentrated under reduced pressure and the residue was purified on a 60g C18 column with a gradient of 50-95% acetonitrile (0.1% formic acid) in water, followed by freeze-drying to give compound 18-2 as a colorless solid (113 mg, 50% yield).

Subsequently, 10% Pd/C (100 mg) was added to a solution of 4-OMePh-AlaGlnThr(OBn)GlyThr(OBn)GlyLys(Cbz)Thr(OBn)OBn (compound 18-2; 108 mg, 77.6 µmol) in 2-propanol (20 mL) and 2M hydrochloric acid (aqueous, 1.0 mL). The mixture was stirred under a hydrogen atmosphere (balloon) for 18 hours. The mixture was then filtered through a 0.45 µm syringe filter. The obtained filtrate was concentrated under reduced pressure, and the residue was dissolved in water and freeze-dried. The dried substance was purified on a 30g C18 column using a gradient of 5-30% acetonitrile (0.1% formic acid) in water, followed by freeze-drying to give compound 19-2 as a colorless solid (7.0 mg, 10% yield).

The ¹H NMR data for compound 19-2 (4-MeOPh-AQTGTGKT) weas measured as follows:
¹H NMR (400 MHz; D2O): δ= 7.76-7.71 (m, 2H), 7.02-6.98 (m, 2H), 4.42-4.28 (m, 5H), 4.24-4.13 (m, 3H), 4.09 (d, J 3.9 Hz, 1H), 4.01-3.96 (m, 2H), 3.89 (s, 2H), 3.81 (s, 3H), 2.91 (t, J 7.4 Hz, 2H), 2.34 (t, J 7.6 Hz, 2H), 2.14-2.06 (m, 1H), 2.00-1.91 (m, 1H), 1.85-1.75 (m, 1H), 1.71-1.54 (m, 3H), 1.42 (d, J 7.2 Hz, 3H), 1.40-1.25 (m, 3H), 1.16-1.07 (m, 8H), 16 exchangeable protons not visible.

### 1.7.3. Synthesis of 2-PhPh-AQTGTGKT

Compound 19-3 (2-PhPh-AQTGTGKT) was synthesized by reacting compound 17-3, which was obtained according to Scheme 12 below, with compound 14 in accordance with Scheme 13, thus resulting in the final target compound, 2-PhPh-AQTGTGKT.

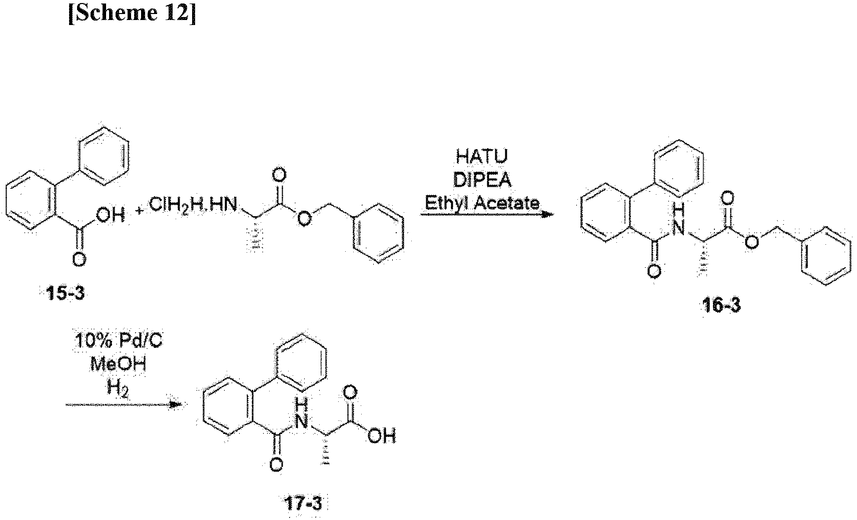

More specifically, H-Ala-OBzl.HCl (388 mg, 1.80 mmol, 1.2 equivalent) was suspended in ethyl acetate (10 mL), and *N,N-*Diisopropylethylamine (653 µL, 3.75 mmol, 2.5 equivalent) was added. After stirring at room temperature for 5 minutes, HATU (855 mg, 2.25 mmol, 1.5 equivalent) and [1,1'-biphenyl]-2-carboxylic acid (297 mg, 1.50 mmol, 1 equivalent) were added, and the mixture was stirred at room temperature for 2 hours. The reaction mixture was then diluted with ethyl acetate and washed with saturated aqueous NH₄Cl, saturated aqueous NaHCO₃, and brine. The obtained organic phase was dried (Na₂SO₄), filtered, and concentrated under reduced pressure. The residue was purified on a 25 g column with a gradient of 2-40% ethyl acetate in heptane to give compound 16-3 as a colorless oil (416 mg, 77% yield).

Next, a minimal amount of water-wet 10% Pd/C (42 mg) was added to a solution of 2-PhPh-AlaOBn (compound 16-3; 416 mg, 1.16 mmol) in methanol (20 mL). The mixture was stirred under a hydrogen atmosphere (balloon) for 18 hours. The mixture was then filtered through a Celite pad, washed with methanol, and the filtrate was concentrated under reduced pressure to give compound 17-3 as a colorless oil (308 mg, 99% yield). The compound 17-3 was then reacted with the compound 14 according to Scheme 13 below.

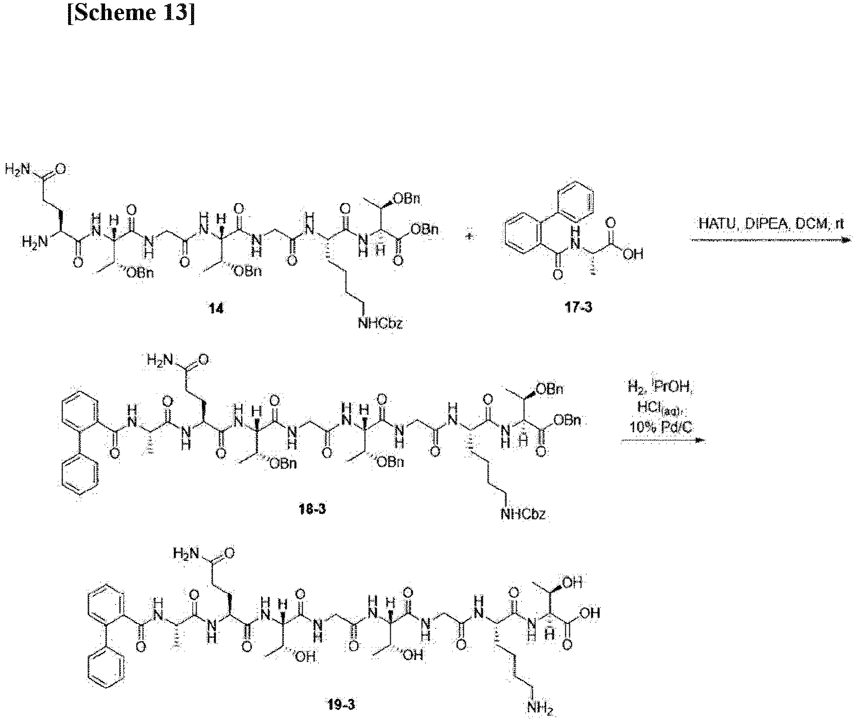

More specifically, *N,N-*Diisopropylethylamine (63.0 µL, 0.360 mmol, 2.2 equivalent) was added to a suspension of GlnThr(OBn)GlyThr(OBn)GlyLys(Cbz)Thr(OBn)OBn (compound 14; 200 mg, 0.164 mmol, 1 equivalent) and 2-PhPh-AlaOH (compound 17-3; 44.2 mg, 0.164 mmol, 1 equivalent) in dichloromethane (20 mL), followed by addition of HATU (74.5 mg, 0.196 mmol, 1.2 equivalent). The mixture was stirred at room temperature for 64 hours, and the reaction mixture was then diluted with methanol, and washed with saturated aqueous NH₄Cl, saturated aqueous NaHCO₃, and water. The organic layer was concentrated under reduced pressure, and the residue was purified on a 60g C18 column with a gradient of 50-95% acetonitrile (0.1% formic acid) in water, followed by freeze-drying to give compound 18-3 as a colorless solid (115 mg, 49% yield).

Subsequently, 10% Pd/C (100 mg) was added to a solution of 2-PhPh-AlaGlnThr(OBn)GlyThr(OBn)GlyLys(Cbz)Thr(OBn)OBn (compound 18-3; 110 mg, 76.5 µmol) in 2-propanol (20 mL) and 2M hydrochloric acid (aqueous, 1.0 mL). The mixture was stirred under a hydrogen atmosphere (balloon) for 18 hours. The mixture was then filtered through a 0.45 µm syringe filter. The filtrate was concentrated under reduced pressure, and the residue was dissolved in water, followed by freeze-drying. The dried substance was purified on a 30 g C18 column with a gradient of 5-50% acetonitrile (0.1% formic acid) in water, and after freeze-drying, compound 19-3 was obtained as a colorless solid (22.7 mg, 31% yield).

The ¹H NMR data for compound 19-3 (2-PhPh-AQTGTGKT) was measured as follows:
¹H NMR (400 MHz; D2O): δ= 7.56-7.48 (m, 2H), 7.44-7.33 (m, 7H), 4.38-4.26 (m, 4H), 4.25-4.13 (m, 4H), 4.08 (d, J 3.9 Hz, 1H), 4.00-3.96 (m, 2H), 3.89 (s, 2H), 2.91 (t, J 7.5 Hz, 2H), 2.25 (t, J 7.5 Hz, 2H), 2.09-2.01 (m, 1H), 1.93-1.77 (m, 2H), 1.72-1.56 (m, 3H), 1.41-1.29 (m, 2H), 1.18 (d, J 7.2 Hz, 3H), 1.12 (d, J 5.6 Hz, 6H), 1.08 (d, J 6.4 Hz, 3H), 16 exchangeable protons not visible.

### 1.7.4. Synthesis of Ph-AQTGTGKT

Ph-AQTGTGKT (compound 19-4) was synthesized by reacting compound 17-4, obtained according to Scheme 14 below, with the compound 14 according to Scheme 15 to ultimately produce the target compound Ph-AQTGTGKT.

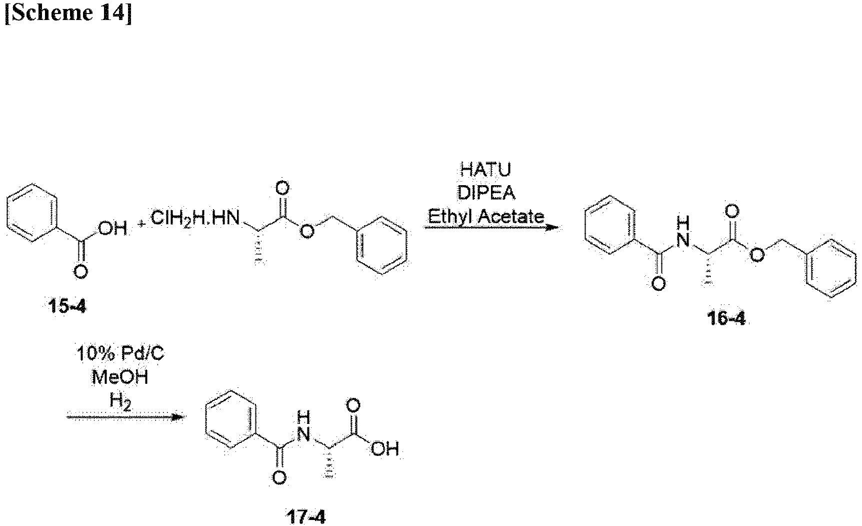

More specifically, H-Ala-OBzl.HCl (388 mg, 1.80 mmol, 1.2 equivalent) was suspended in ethyl acetate (10 mL), and *N,N-*Diisopropylethylamine (653 µL, 3.75 mmol, 2.5 equivalent) was added. After stirring at room temperature for 5 minutes, HATU (855 mg, 2.25 mmol, 1.5 equivalent) and benzoic acid (183 mg, 1.50 mmol, 1 equivalent) were added, and the mixture was stirred at room temperature for 18 hours. The reaction mixture was diluted with ethyl acetate and washed with saturated aqueous NH₄Cl, saturated aqueous NaHCO₃, and brine. The obtained organic phase was dried (Na₂SO₄), filtered, and concentrated under reduced pressure. The residue was purified on a 25 g column with 2-50% ethyl acetate in heptane to give compound 16-4 as a colorless oil (375 mg, 88% yield).

Subsequently, a minimal amount of water-wet 10% Pd/C (38 mg) was added to a solution of Ph-Ala-OBn (compound 16-4; 375 mg, 1.32 mmol) in methanol (30 mL). The mixture was stirred under a hydrogen atmosphere (balloon) for 4 hours. The mixture was then filtered through a Celite pad and washed with methanol. The obtained filtrate was concentrated under reduced pressure to give compound 17-4 as a colorless glass (glass; 254 mg, 99% yield), which was reacted with compound 14 according to Scheme 15 below.

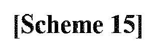

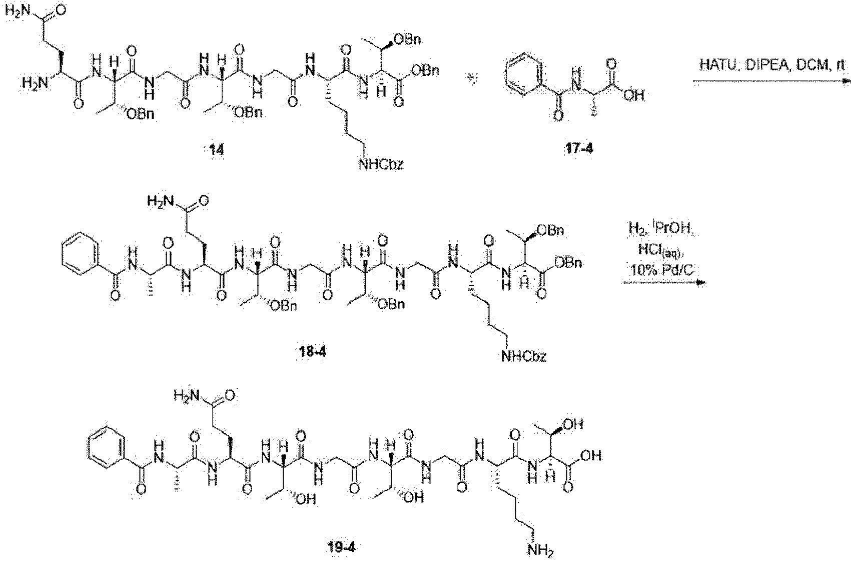

More specifically, HATU (106 mg, 0.278 mmol, 1.1 equivalent) was added to a suspension of GlnThr(OBn)GlyThr(OBn)GlyLys(Cbz)Thr (OBn)OBn (compound 14; 300 mg, 0.253 mmol, 1 equivalent) and Ph-Ala-OH (compound 17-4; 49.0 mg, 0.253 mmol, 1 equivalent) in dichloromethane (20 mL) and *N,N-*Diisopropylethylamine (97.0 µL, 0.556 mmol, 2.2 equivalent). The mixture was stirred at room temperature for 2 hours and the reaction mixture was washed with saturated aqueous NaHCO₃. The organic phase was concentrated under reduced pressure and the obtained residue was purified on a 60g C18 column with 40-100% acetonitrile (0.1% formic acid) in water, followed by freeze-drying to give compound 18-4 as a colorless solid (200 mg, 58%).

Subsequently, 10% Pd/C (110 mg) was added to a solution of Ph-AlaGlnThr(OBn)GlyThr(OBn)GlyLys(Cbz)Thr(OBn)OBn (compound 18-4; 110 mg, 80.8 µmol) in 2-propanol (20 mL) and 2M aqueous HCl (1.0 mL). The mixture was stirred under a hydrogen atmosphere (balloon) for 18 hours. The mixture was then filtered through a 0.45 µm syringe filter. The obtained filtrate was concentrated under reduced pressure, and the residue was dissolved in water and freeze-dried. The dried material was purified on a 60g C18 column using a gradient of 5-50% acetonitrile (0.1% formic acid) in water, followed by freeze-drying to give compound 19-4 as a colorless solid (23.2 mg, 33% yield).

The ¹H NMR data for compound 19-4 (Ph-AQTGTGKT) was measured as follows:
¹H NMR (400 MHz; D2O): δ= 7.74-7.70 (m, 2H), 7.58-7.53 (m, 1H), 7.48-7.43 (m, 2H), 4.45-4.34 (m, 3H), 4.32-4.27 (m, 2H), 4.25-4.14 (m, 3H), 4.11 (d, J 3.8 Hz, 1H), 4.00-3.96 (m, 2H), 3.89 (s, 2H), 2.91 (t, J 7.4 Hz, 2H), 2.34 (t, J 7.6 Hz, 2H), 2.14-2.06 (m, 1H), 2.01-1.91 (m, 1H), 1.85-1.76 (m, 1H), 1.71-1.56 (m, 3H), 1.43 (d, J 7.3 Hz, 3H), 1.40-1.30 (m, 2H), 1.16-1.07 (m, 9H), 16 exchangeable protons not visible.

### 1.7.5. Synthesis of Naphthyl-AQTGTGKT

Naphthyl-AQTGTGKT (compound 19-5) was synthesized by reacting compound 17-5, obtained according to Scheme 16 below, with the compound 14 according to Scheme 17, to produce the final target compound, Naphthyl-AQTGTGKT.

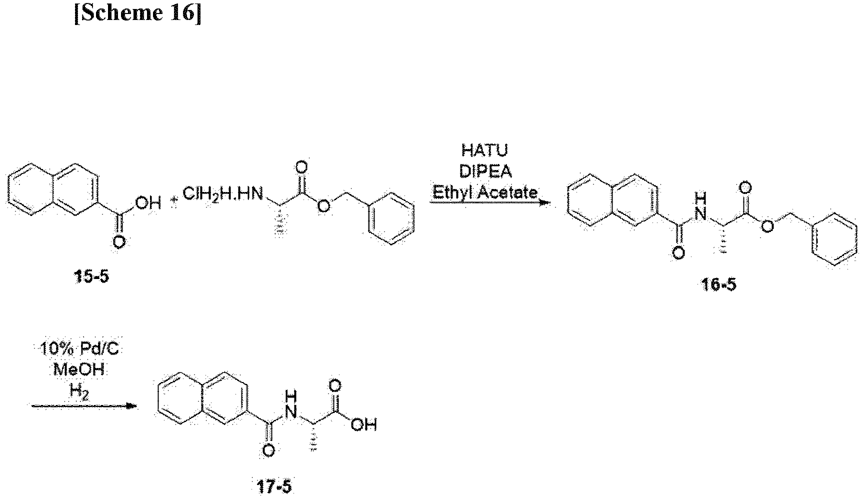

More specifically, H-Ala-OBzl.HCl (388 mg, 1.80 mmol, 1.2 equivalent) was suspended in ethyl acetate (10 mL) and *N,N-*Diisopropylethylamine (653 µL, 3.75 mmol, 2.5 equivalent) was added. After stirring at room temperature for 5 minutes, HATU (855 mg, 2.25 mmol, 1.5 equivalent) and 2-naphthoic acid (258 mg, 1.50 mmol, 1 equivalent) were added and the mixture was stirred at room temperature for 2 hours. The reaction mixture was diluted with ethyl acetate, then washed with saturated aqueous NH₄Cl, saturated aqueous NaHCO₃ and saline. The resulting organic matter was dried (Na₂SO₄), filtered and concentrated under reduced pressure. The residue was purified on a 25 g column with 2-40% ethyl acetate in heptane to give compound 16-5 as a colorless solid (385 mg, 77% yield).

Subsequently, a minimum amount of water-wet 10% Pd/C (39 mg) was added to a solution of 2-Naphthyl-AlaOBn (compound 16-5; 385 mg, 1.15 mmol) in methanol (20 mL). The mixture was stirred under a hydrogen atmosphere (balloon) for 4 hours. The mixture was then filtered through a Celite pad and washed with methanol. The obtained filtrate was concentrated under reduced pressure to give compound 17-5 as a colorless solid (266 mg, 95% yield), which was then reacted with compound 14 according to Scheme 17 below.

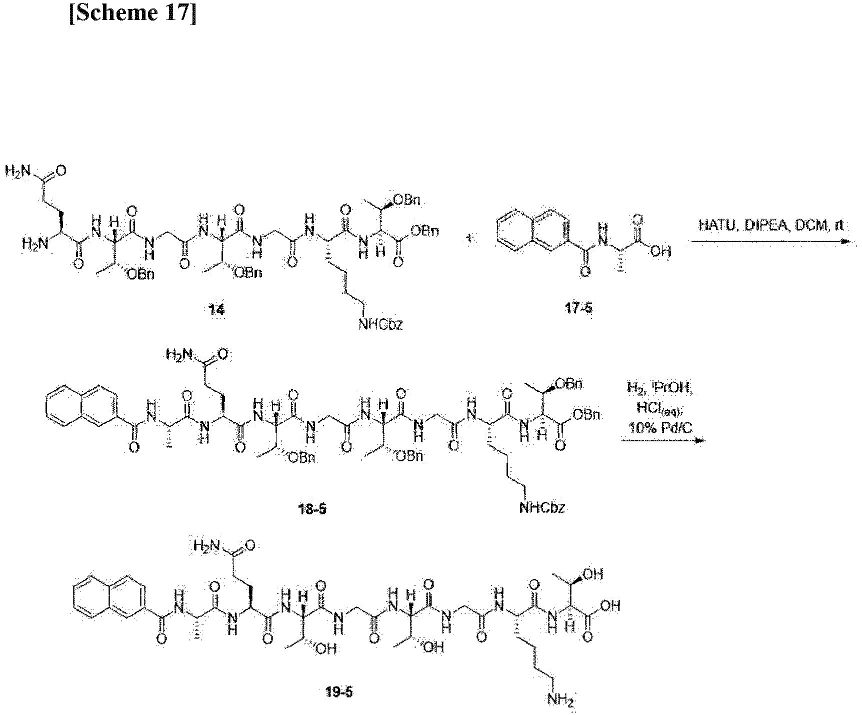

More specifically, *N,N-*Diisopropylethylamine (97.0 µL, 0.556 mmol, 2.2 equivalent) and a suspension of GlnThr(OBn)GlyThr(OBn)GlyLys(Cbz)Thr(OBn)OBn (compound 14; 300 mg, 0.253 mmol, 1 equivalent) and 2-Naphthyl-Ala-OH (compound 17-5; 61.0 mg, 0.253 mmol, 1 equivalent) in dichloromethane (20 mL) were added to HATU (106 mg, 0.278 mmol, 1.1 equivalent). The mixture was stirred at room temperature for 2 hours and then washed with saturated aqueous NaHCO₃. The obtained organic layer was concentrated under reduced pressure and the residue was purified on a 60g C18 column with a gradient of 40-100% acetonitrile (0.1% formic acid) in water. After freeze-drying, compound 18-5 was obtained as a colorless solid (230 mg, 64% yield).

Subsequently, 10% Pd/C (101 mg) was added to a solution of 2-Naphthyl-AlaGlnThr(OBn)GlyThr(OBn)GlyLys(Cbz)Thr(OBn)OBn (compound 18-5; 101 mg, 71.5 µmol) in 2M HCl (aqueous, 1.0 mL) and 2-propanol (20 mL). The mixture was stirred under a hydrogen atmosphere (balloon) for 3 hours. The mixture was then filtered through a 0.45 µm syringe filter. The obtained filtrate was concentrated under reduced pressure, and the residue was dissolved in water and then freeze-dried. The dried material was purified on a 30 g C18 column with a gradient of 5-40% acetonitrile (0.1% formic acid) in water. After freeze-drying, compound 19-5 was obtained as a colorless solid (23.2 mg, 33% yield).

The ¹H NMR data for compound 19-5 (Naphthyl-AQTGTGKT) was measured as follows:
¹H NMR (400 MHz; D2O): δ= 8.32 (s, 1H), 8.01-7.90 (m, 3H), 7.79-7.73 (m, 1H), 7.64-7.55 (m, 2H), 4.51-3.70 (m, 13H), 2.96-2.81 (m, 2H), 2.39-2.30 (m, 2H), 2.18-2.04 (m, 1H), 2.03-1.93 (m, 1H), 1.85-1.72 (m, 1H), 1.71-1.53 (m, 3H), 1.50-1.43 (m, 3H), 1.39-1.24 (m, 2H), 1.19-1.04 (m, 9H), 16 exchangeable protons not visible.

### 1.8. Synthesis of Ac-AQTGTGKT

Ac-AQTGTGKT (compound 19-6) was synthesized by performing the process according to Scheme 18 below to produce the final target compound Ac-AQTGTGKT.

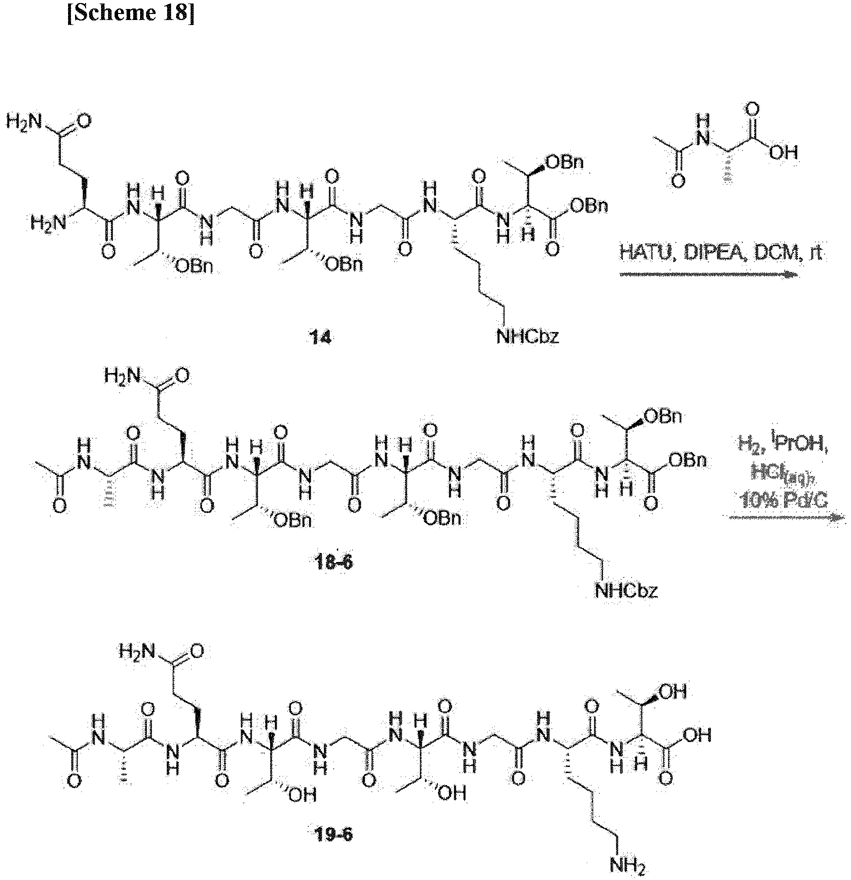

More specifically, HATU (112 mg, 0.294 mmol, 1.2 equivalent) was added to a suspension of GlnThr(OBn)GlyThr(OBn)GlyLys(Cbz)Thr(OBn)OBn (compound 14; 300 mg, 0.245 mmol, 1 equivalent) and Ac-Ala-OH (32.1 mg, 0.245 mmol, 1 equivalent) in *N,N*-Diisopropylethylamine (94.0 µL, 0.540 mmol, 2.2 equivalent) and dichloromethane (30 mL). The mixture was stirred at room temperature for 14 hours, and the mixture was washed with saturated aqueous NH₄Cl, NaHCO₃, and water. The organic layer was concentrated under reduced pressure, and the residue was purified on a 60g C18 column with a gradient of 50-95% acetonitrile (0.1% formic acid) in water (0.1% formic acid). The desired fractions were combined and freeze-dried to give compound 18-6 as a colorless solid (104 mg, 33% yield).

Subsequently, 10% Pd/C (10 mg) was added to a solution of Ac-AlaGlnThr(OBn)GlyThr(OBn)GlyLys(Cbz)Thr(OBn)OBn (compound 18-6; 33 mg, 25 µmol) in 2M hydrochloric acid (aqueous, 0.19 mL) and 2-propanol (5 mL). The mixture was stirred under a hydrogen atmosphere (balloon) for 14 hours. The mixture was filtered through a 0.45 µm syringe filter. The resulting filtrate was concentrated under reduced pressure, dissolved in water, and then freeze-dried. The dried material was eluted with 0.5M ammonia in methanol through a 500 mg SCX-2 cartridge. The desired fractions were combined and concentrated under reduced pressure, then freeze-dried to give compound 19-6 as a colorless solid (7.6 mg, 37% yield).

The ¹H NMR data for compound 19-6 (Ac-AQTGTGKT) was measured as follows: ¹H NMR (400 MHz; D2O): δ= 4.40-4.33 (m, 2H), 4.32-4.27 (m, 2H), 4.24-4.12 (m, 4H), 4.08 (d, J 4.0 Hz, 1H), 4.03-3.88 (m, 4H), 2.92 (t, J 7.2 Hz, 2H), 2.32 (t, J 7.8 Hz, 2H), 2.15-2.02 (m, 1H), 1.99-1.88 (m, 4H), 1.86-1.76 (m, 1H), 1.74-1.55 (m, 3H), 1.45-1.31 (m, 2H), 1.29 (t, J 7.4 Hz, 2H), 1.20-1.06 (m, 10H), 16 exchangeable protons not visible.

### 1.9. Synthesis of 4-PhPh-AQTGTGKT

### 1.9.1. Synthesis of QTGTGKT Intermediate

QTGTGKT intermediate for the synthesis of 4-PhPh-AQTGTGKT was synthesized according to following Schemes 19 to 23:

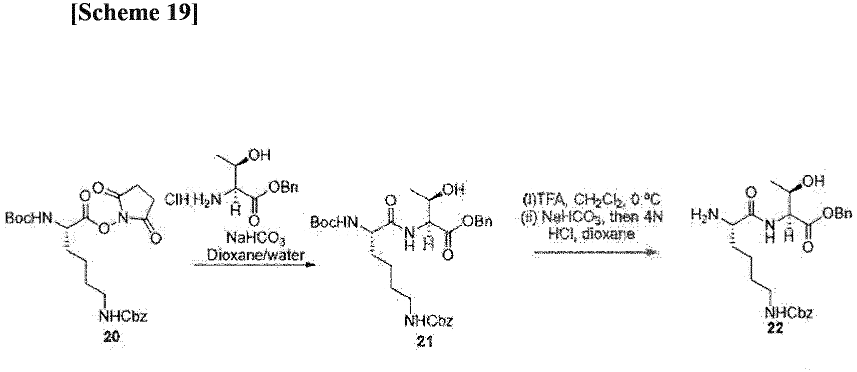

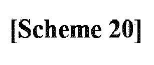

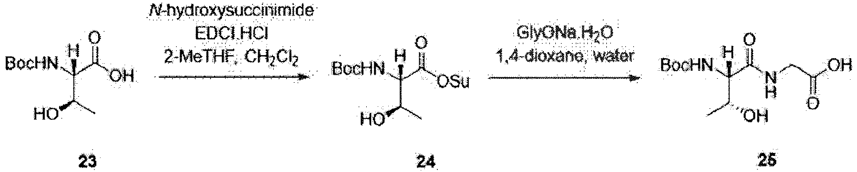

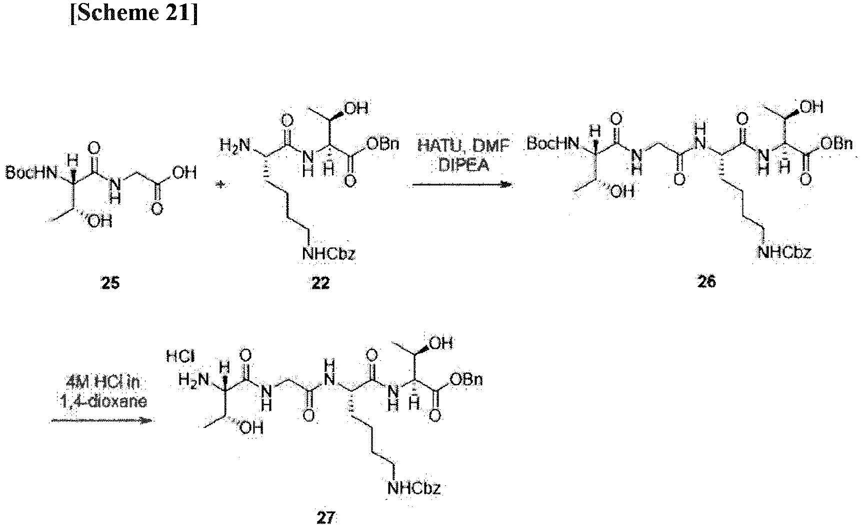

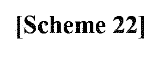

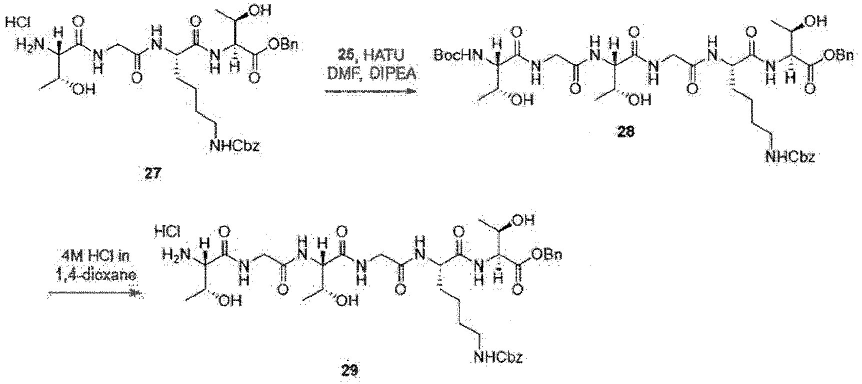

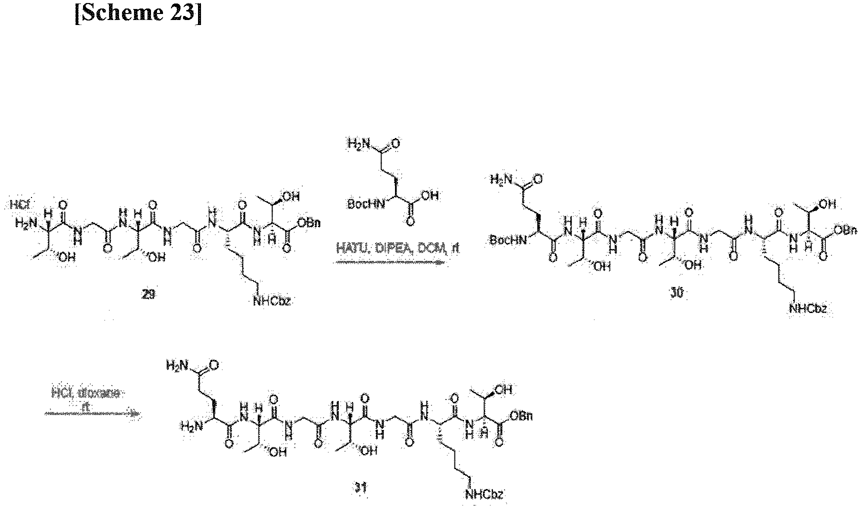

Since Schemes 19 to 23 are almost similar processes to Schemes 1 to 5, excluding the presence or absence of the benzyl protecting group, redundant descriptions are omitted.

### 1.9.2. Synthesis of 4-PhPh-AQTGTGKT

The compound 31 obtained from Scheme 23 was combined with an alanine derivative synthesized in Scheme 24 according to Scheme 25 to obtain the final product 4-PhPh-AQTGTGKT.

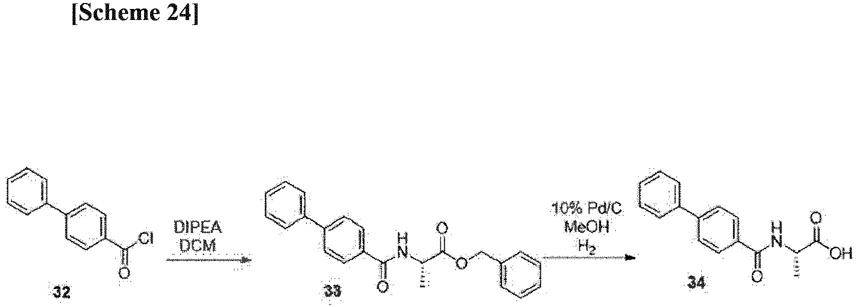

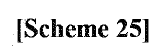

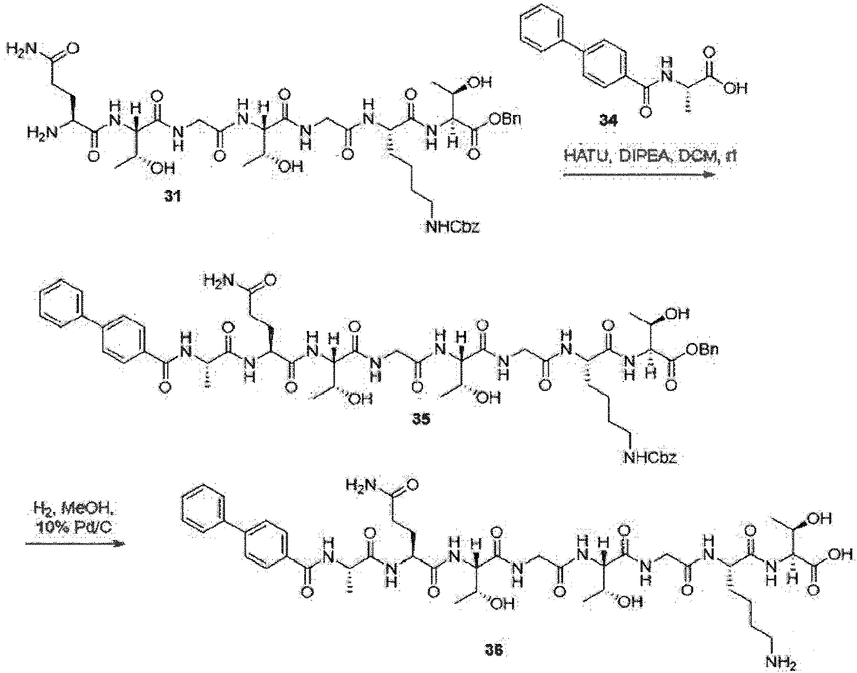

Since Schemes 24 and 25 were also conducted by almost similar processes to the aforementioned schemes, redundant descriptions are omitted. Finally, compound 36 was obtained as a colorless solid (583 mg, 68% yield).

The ¹H NMR data for compound 36 (4-PhPh-AQTGTGKT) was measured as follows:
¹H NMR (400 MHz; D2O): δ= 7.85 (d, J 8.8 Hz, 2H), 7.77 (d, J 8.8 Hz, 2H), 7.70 (d, J 7.2 Hz, 2H), 7.49 (t, J 7.2 Hz, 2H), 7.42 (t, J 7.1 Hz, 1H), 4.34-4.04 (m, 6H), 4.07 (d, J 3.6 Hz, 1H), 3.93 (d, J 2.0 Hz, 2H), 2.82 (t, J 7.0 Hz, 2H), 1.82-1.67 (m, 1H), 1.66-1.55 (m, 1H), 1.54-1.44 (m, 2H), 1.37-1.22 (m, 2H), 1.18 (d, J 6.4 Hz, 3H), 1.06 (t, J 6.5 Hz, 3H), 10 exchangeable protons not visible.

### 1.10. Compound Verification

The 13 analogs of AQTGTGKT obtained by the methods above were analyzed by ¹H NMR and UPLC-MS (ultraperformance liquid chromatography-mass spectrometry) techniques to confirm their chemical structures.

The structural formulas for the 13 kinds of AQTGTGKT analog compounds are presented in Table 1 below.

**[Table 1]**

| **Name** | **Formula** |
|---|---|
| 4-PhPh-AQTGTGKT | 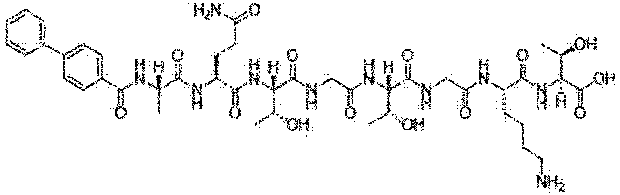 |
| Ac-AQTGTGKT | 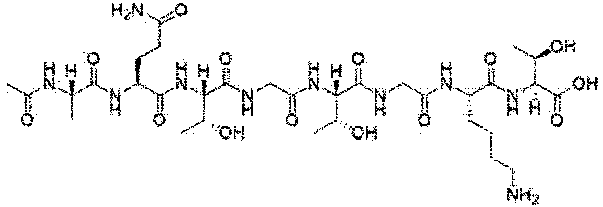 |
| 3-PhPh-AQTGTGKT | 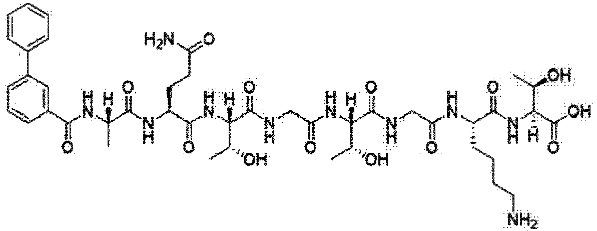 |
| 4-MeOPh-AQTGTGKT | 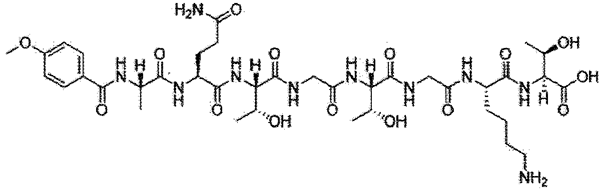 |
| 2-PhPh-AQTGTGKT | 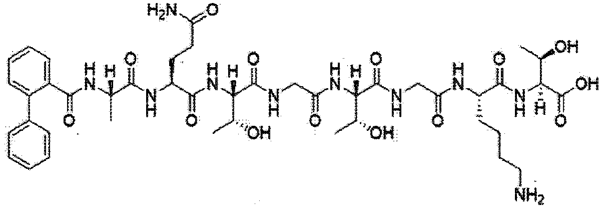 |
| Ph-AQTGTGKT | 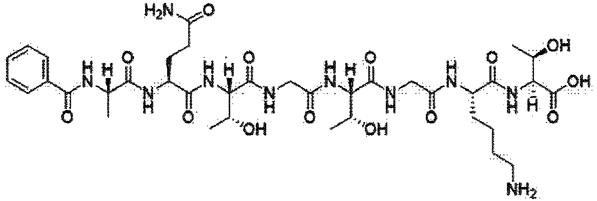 |
| Naphthyl-AQTGTGKT | 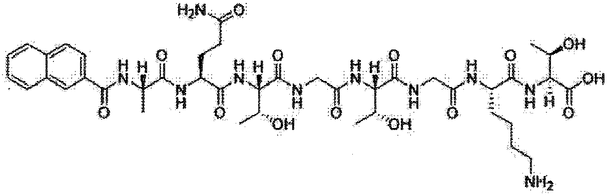 |
| 2,4,6-MePh-AQTGTGKT | 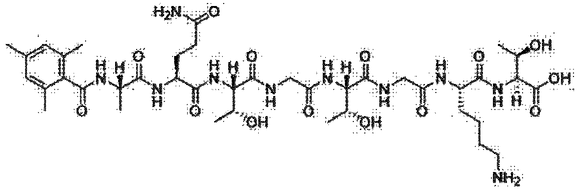 |
| 1-MeOCF₃Ph-QTGTGKT | 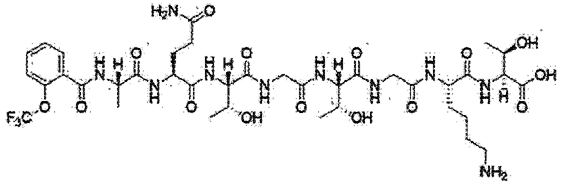 |
| 4-MorPh-AQTGTGKT | 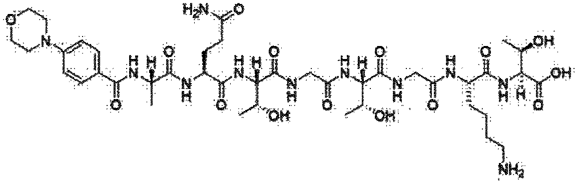 |
| PhMeMeC-AQTGTGKT | 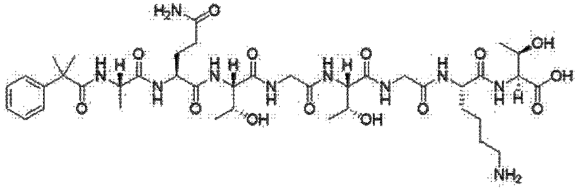 |
| 4-CF₃Ph-AQTGTGKT | 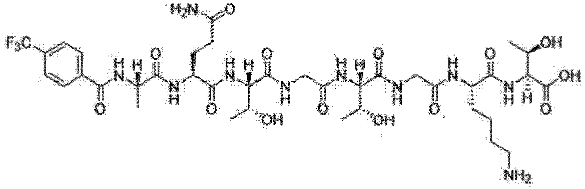 |
| Ph₃C-AQTGTGKT | 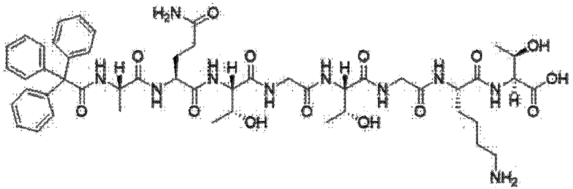 |

### 2. MTT (tetrazolium) assay

Compounds according to the present invention were treated to cancer cell lines, and the effect of co-administration was measured as cell viability degree through MTT assay by adding a targeted anticancer drug (Osimertinib or Olaparib) or a chemotherapeutic agent (Alimta or Cisplatin) simultaneously or after 4 hours. Specifically, cells were seeded at 2 × 10³/100 µl per well on a 96-well plate, and after culturing for 24 hours, the compound according to the present invention was administered; 4 hours later, anticancer drugs were treated to each cancer cell. The treatment concentration and treatment time of the compound and anticancer drug are specifically described in the embodiment. After completing the treatment of the compound and anticancer drug, CellTiter-96^{®} reagent (Promega Co., USA) was reacted at 5% CO₂, 37°C after adding 10 µl per well. After 3 hours, absorbance was measured at 490 nm using a spectrophotometer (SPECTROstar^{Nano}, BMG).

### 3. Heterotransplant lung cancer animal model

H820 cells were mixed with matrigel in a 1:1 ratio at a concentration of 5 × 10⁶ cells/mouse, and each was subcutaneously inoculated at 200 µl into the flank of the mouse once. After the inoculation was completed, it was confirmed that the volume of the formed tumor reached 70-130 mm³, and random grouping was then carried out. The test substances were administered intravenously and orally 7 times at 2-day intervals.

### 4. Heterotransplant lung cancer resistance animal model

H1975 cells were mixed with matrigel in a 1:1 ratio at a concentration of 5 × 10⁶ cells/mouse, and each was subcutaneously inoculated at 200µl into the flank of the mouse once. After the inoculation, it was confirmed that the volume of the formed tumor reached 70~130 mm³, and then random grouping was carried out. After the grouping, Osimertinib was administered orally every day, and after the growth of the tumor was inhibited, the point of re-growth was identified and the co-administration with the test substance was started. The test substances were administered intravenously 7 times at 2-day intervals.

### 5. Immunohistochemical staining (IHC)

Tissue embedded in paraffin was cut to a thickness of 4 µm and affixed to a slide, then thoroughly dried and the IHC experiment procedure was performed. Each slide was deparaffinized, and endogenous enzymes were removed using citrate buffer (pH 6.0). Then, p-Beclin1S15 antibody was diluted to 1:100 and treated for 15 hours at 4°C. After washing 4 times with TBST wash buffer, rabbit HRP was treated for secondary antibody reaction and reacted at room temperature for 30 minutes. After the substrate for the antibody reaction was added, when the color appeared, the slide was washed and sealed, and after slide scanning, the result was read.

### Example 1: Anticancer Effect of Co-administration of Targeted Anticancer Drug/Chemotherapeutic Agent with AQTGTGKT or its Analogs in Lung Cancer Cells

### 1.1. Effect of Co-administration in Lung Cancer Cell Line H1975

The anticancer effect of combination of AQTGTGKT or its analogs in lung cancer cell line H1975 was confirmed in accordance with the MTT assay method described in "Experimental Materials and Methods" above.

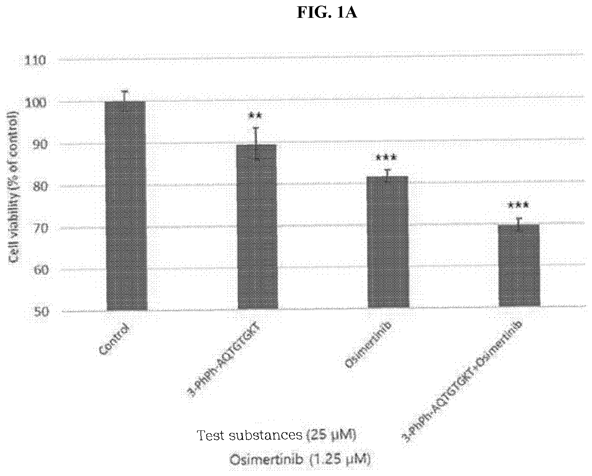

Initially, the effect of the combined treatment with Osimertinib, a targeted anticancer drug, was verified. H1975 was treated with 3-PhPh-AQTGTGKT at a concentration of 25 µM and after 4 hours, Osimertinib was administered at a concentration of 1.25 µM, either alone or in combination, and the cell viability inhibitory effects were compared 68 hours later. As shown in FIG. 1A, when treated with 3-PhPh-AQTGTGKT alone, cell viability decreased by about 10% compared to the control group (untreated control, hereinafter the same). When Osimertinib was administered alone, cell viability decreased by about 19% compared to the control group. On the other hand, the combined treatment of 3-PhPh-AQTGTGKT and Osimertinib resulted in approximately 30% cell viability inhibition.

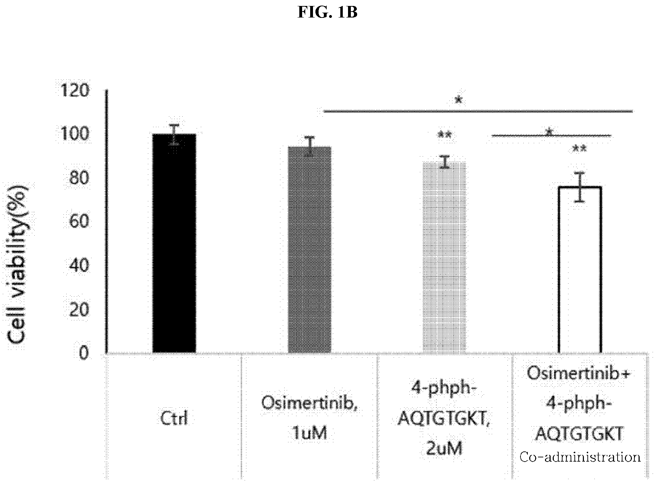

Subsequently, H1975 was treated with 4-PhPh-AQTGTGKT at a concentration of 2 µM, and at the same time, Osimertinib was administered at a concentration of 1 µM, either alone or in combination, and the cell viability inhibitory effects were compared 48 hours later. As a result, when 4-PhPh-AQTGTGKT was administered alone, cell viability decreased by about 12% compared to the control group. When Osimertinib was administered alone, cell viability decreased by about 5% compared to the control group. On the other hand, the combined treatment of 4-PhPh-AQTGTGKT and Osimertinib resulted in approximately 24% cell viability inhibition (FIG. 1B).

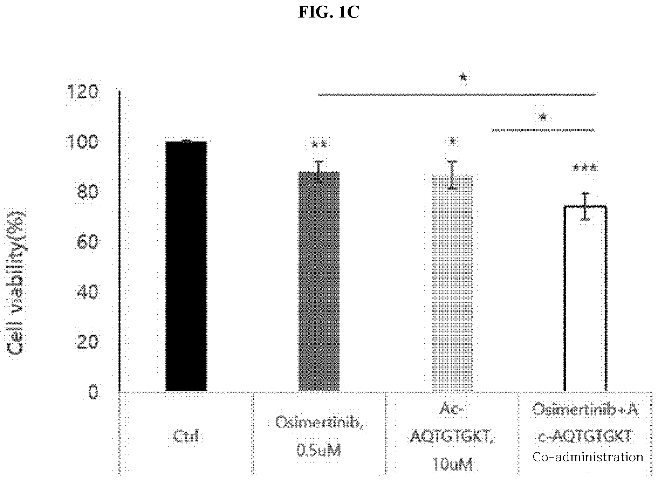

Next, H1975 was treated with Ac-AQTGTGKT at a concentration of 10 µM, and at the same time, Osimertinib was administered at a concentration of 0.5 µM, either alone or in combination, and the cell viability inhibitory effects were compared 48 hours later. As a result, when Ac-AQTGTGKT was administered alone, cell viability decreased by about 14% compared to the control group. When Osimertinib was administered alone, cell viability decreased by about 12% compared to the control group. On the other hand, the combined treatment of Ac-AQTGTGKT and Osimertinib resulted in approximately 26% cell viability inhibitory effect (FIG. 1C).

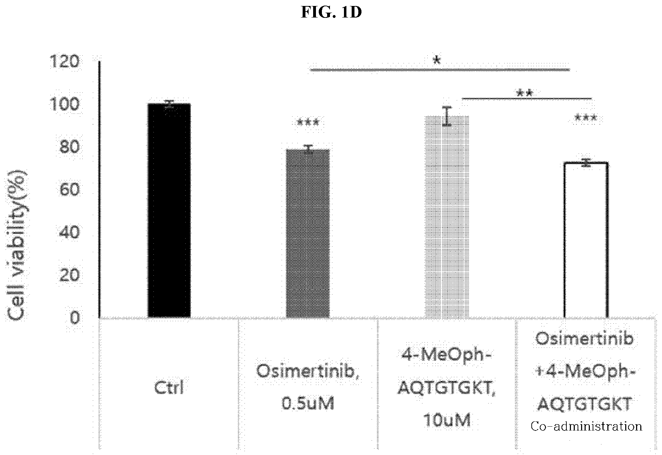

Furthermore, H1975 was treated with 4-MeOPh-AQTGTGKT at a concentration of 10 µM and simultaneously, a targeted anticancer drug, Osimertinib, was administered either alone or in combination at a concentration of 0.5 µM, and after 48 hours, the effects of inhibiting cell viability were compared. As a result, when 4-MeOPh-AQTGTGKT was administered alone, cell viability decreased by about 6% compared to the control group, and when Osimertinib was administered alone, cell viability decreased by about 21% compared to the control group. On the other hand, the combined treatment of 4-MeOPh-AQTGTGKT and Osimertinib resulted in approximately 27% cell viability inhibitory effect (FIG. 1D).

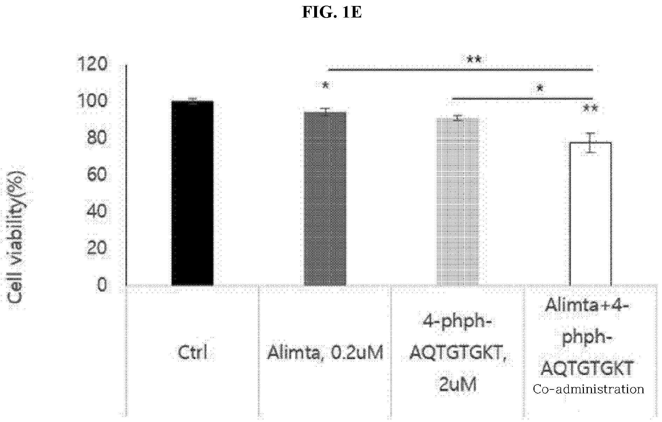

Next, the effect of the combined treatment with the chemotherapeutic agent Alimta (Pemetrexed) was confirmed. Similarly, the lung cancer cell line H1975 was treated with 4-PhPh-AQTGTGKT at a concentration of 2 µM according to the MTT assay method described in the "Experimental Materials and Methods". At the same time, Alimta, a chemotherapeutic agent, was administered either alone or in combination at a concentration of 0.2 µM, and after 48 hours, the effects of inhibiting cell viability were compared. As shown in FIG. 1E, when 4-PhPh-AQTGTGKT was administered alone, cell viability decreased by about 9% compared to the control group, and when Alimta was administered alone, cell viability decreased by about 6% compared to the control group. On the other hand, the combined treatment of 4-PhPh-AQTGTGKT and Alimta resulted in approximately 22% cell viability inhibitory effect.

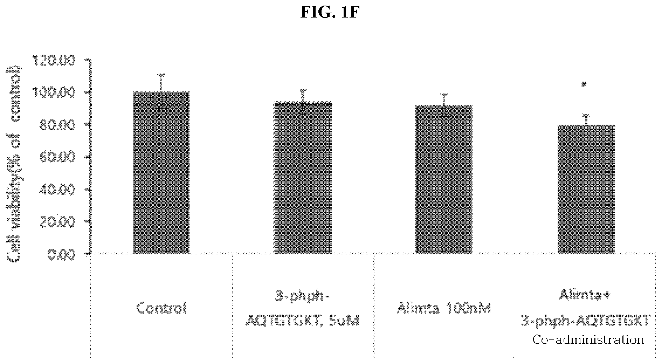

Additionally, H1975/OR was treated with 3-PhPh-AQTGTGKT at a concentration of 5 µM, and after 4 hours, the chemotherapeutic agent Alimta was administered either alone or in combination at a concentration of 0.1 µM, and after 72 hours, the effects of inhibiting cell viability were compared. As shown in FIG. 1F, when 3-PhPh-AQTGTGKT was administered alone, cell viability decreased by about 6.5% compared to the control group, and when Alimta was administered alone, cell viability decreased by about 8.3% compared to the control group. On the other hand, the combined treatment of 3-PhPh-AQTGTGKT and Alimta resulted in approximately 20% cell viability inhibitory effect.

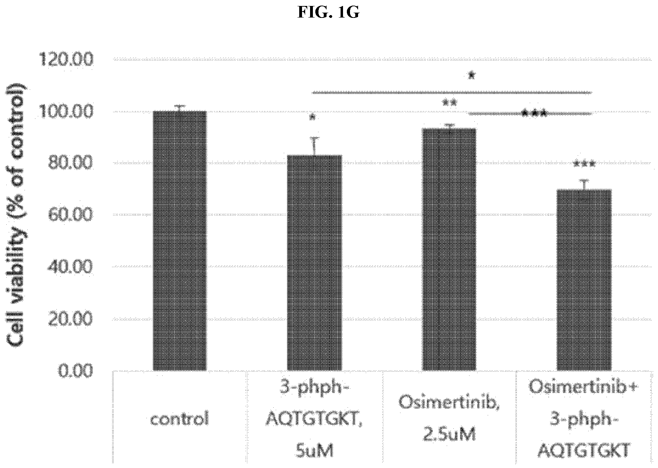

Subsequently, H1975/OR was treated with 3-PhPh-AQTGTGKT at a concentration of 5 µM, and after 4 hours, the targeted anticancer drug Osimertinib was administered either alone or in combination at a concentration of 2.5 µM, and after 68 hours, the effects of inhibiting cell viability were compared. As shown in FIG. 1G, when 3-PhPh-AQTGTGKT was administered alone, cell viability decreased by about 17% compared to the control group, and when Osimertinib was administered alone, cell viability decreased by about 7% compared to the control group. On the other hand, the combined treatment of 3-PhPh-AQTGTGKT and Osimertinib resulted in approximately 30% cell viability inhibitory effect.

In addition, the anticancer effect of a triple therapy with the compound, targeted anticancer drug, and chemotherapeutic agent of the present invention has been confirmed. Specifically, the effect of lung cancer cell death by triple co-administration of 3-PhPh-AQTGTGKT, Osimertinib, and Alimta was confirmed, and at this time, 3-PhPh-AQTGTGKT was administered with a concentration of 5 µM, and Osimertinib and Alimta were each administered with concentrations of 2.5 µM and 100 nM to H1975 cells for 72 hours (respectively, the concentration treated in the anticancer effect confirmation experiment with 3-PhPh-AQTGTGKT). The anticancer effect of the triple co-administration was compared with the 3-PhPh-AQTGTGKT single treatment group, and the Osimertinib and Alimta co-treatment group.

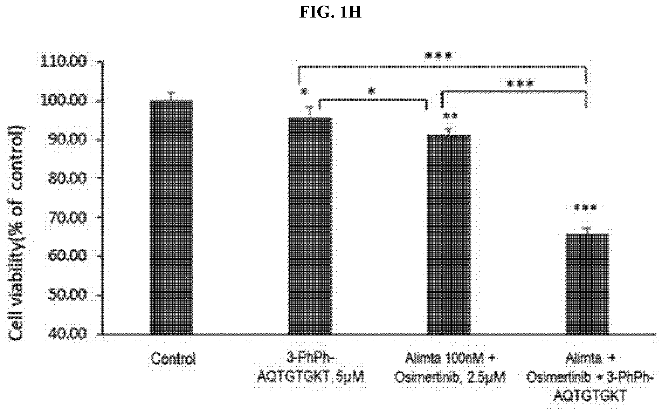

As a result, the triple co-administration group of 3-PhPh-AQTGTGKT, Osimertinib, and Alimta showed a much superior cell viability inhibition efficacy compared to the 3-PhPh-AQTGTGKT single treatment group or the Osimertinib and Alimta co-treatment group, suppressing cell growth by about 35% compared to the control group (FIG. 1H). The results indicate that not only is the anticancer effect more excellent when the compound according to the present invention is co-administered with a targeted anticancer drug or chemotherapeutic agent compared to monotherapy, but also the anticancer effect may be significantly enhanced when triple co-administered with a targeted anticancer drug and chemotherapeutic agent.

### 1.2. Effect of Co-administration in Lung Cancer Cell Line H820

The anticancer effect of combination with AQTGTGKT or its analogs in the lung cancer cell line H820 was confirmed according to the MTT assay method described in "Experimental Materials and Methods".

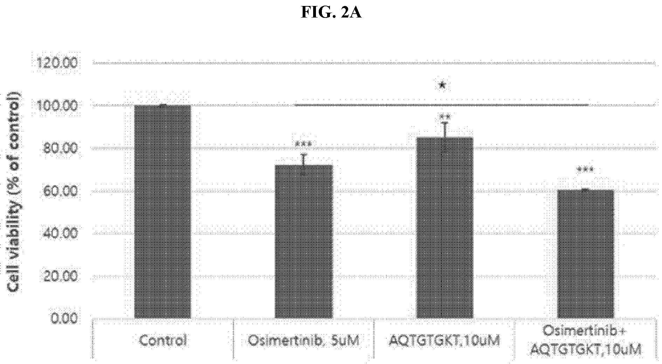

First, AQTGTGKT was administered at a concentration of 10 µM to H820, and then, the targeted anticancer drug Osimertinib was administered either alone or in combination at a concentration of 5 µM, and the cell viability inhibitory effect was compared 48 hours later. As a result, as shown in FIG. 2A, when AQTGTGKT was administered alone, cell viability decreased by about 15% compared to the control group, and when Osimertinib was administered alone, cell viability decreased by about 28% compared to the control group. In contrast, the combined treatment of AQTGTGKT and Osimertinib resulted in approximately 40% cell viability inhibitory effect.

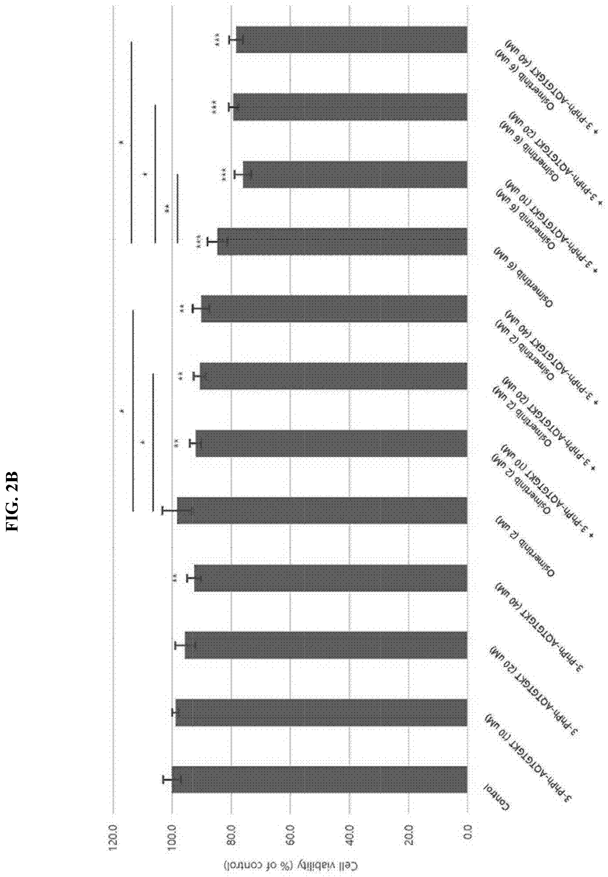

Next, 3-PhPh-AQTGTGKT was administered at a concentration of 40 µM to H820, and then, the targeted anticancer drug Osimertinib was administered either alone or in combination at a concentration of 6 µM, and the cell viability inhibitory effect was compared 72 hours later. As a result, as shown in FIG. 2B, when 3-PhPh-AQTGTGKT was administered alone, cell viability decreased by about 8% compared to the control group, and when Osimertinib was administered alone, cell viability decreased by about 15% compared to the control group. In contrast, the combined treatment of 3-PhPh-AQTGTGKT and Osimertinib resulted in approximately 22% cell viability inhibitory effect.

### 1.3. Effect of Co-administration in Lung Cancer Cell Line PC9/OR

In accordance with the MTT assay method described in the "Experimental Materials and Methods", the non-small cell lung cancer cell line PC9/OR was treated with 3-PhPh-AQTGTGKT at a concentration of 25 µM, and 4 hours later, the targeted anticancer drug Osimertinib was administered either alone or in combination at a concentration of 4 µM, and the cell toxicity was compared 68 hours later.

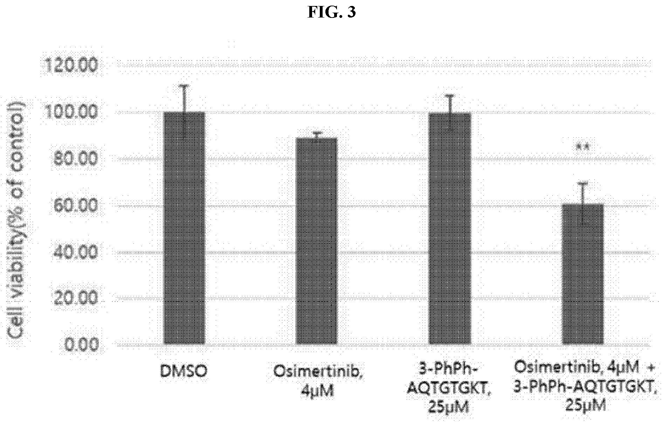

As shown in FIG. 3, when 3-PhPh-AQTGTGKT was administered alone, cell viability decreased by approximately 0.2% compared to the control group. When Osimertinib was administered alone, cell viability decreased by approximately 10% compared to the control group. In contrast, the combined treatment of 3-PhPh-AQTGTGKT and Osimertinib resulted in approximately 40% cell viability inhibitory effect.

The results from Examples 1.1 to 1.3 demonstrate that in lung cancer cells, the combined treatment of AQTGTGKT or its analogs and targeted anticancer drugs according to the present invention is much more effective than the single treatment.

### Example 2: Anticancer Effect of Co-administration of Targeted Anticancer Drug/Chemotherapeutic Agent with AQTGTGKT or its Analogs in Breast Cancer Cells

The anticancer effect of combination of AQTGTGKT or its analogs in breast cancer cell line HCC1937 was confirmed in accordance with the MTT assay method described in "Experimental Materials and Methods" above.

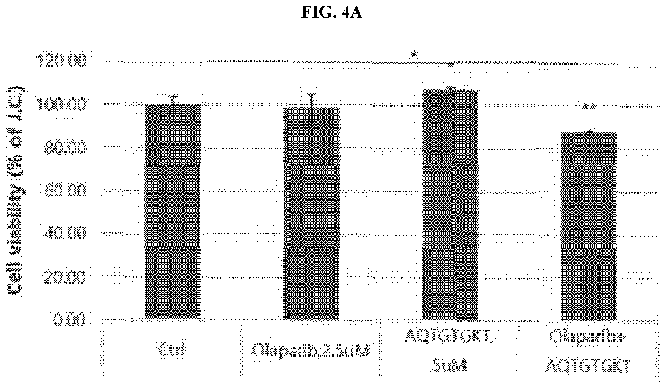

First, the breast cancer cell line HCC1937 was treated with AQTGTGKT at a concentration of 5 µM and simultaneously the targeted anticancer drug Olaparib was administered either alone or in combination at a concentration of 2.5 µM. After 24 hours, the cell viability inhibitory effect was compared. As shown in FIG. 4A, when AQTGTGKT was administered alone, there was no decrease in cell viability compared to the control group, and when Olaparib was administered alone, there was a decrease in cell viability of about 1.2% compared to the control group. In contrast, the combined treatment of AQTGTGKT and Olaparib resulted in approximately 13% cell viability inhibitory effect.

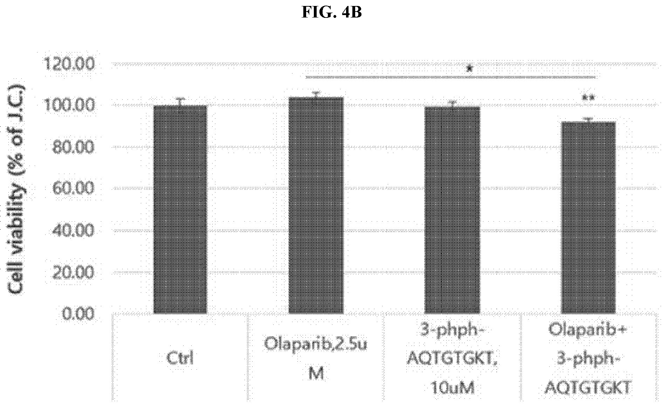

Next, HCC1937 was treated with 3-PhPh-AQTGTGKT at a concentration of 10 µM, and simultaneously the targeted anticancer drug Olaparib was administered either alone or in combination at a concentration of 2.5 µM. After 24 hours, the cell viability inhibitory effect was compared. As shown in FIG. 4B, when 3-PhPh-AQTGTGKT was administered alone, there was no decrease in cell viability compared to the control group, and when Olaparib was administered alone, there was no decrease in cell viability compared to the control group. In contrast, the combined treatment of 3-PhPh-AQTGTGKT and Olaparib resulted in approximately 8.2% cell viability inhibitory effect.

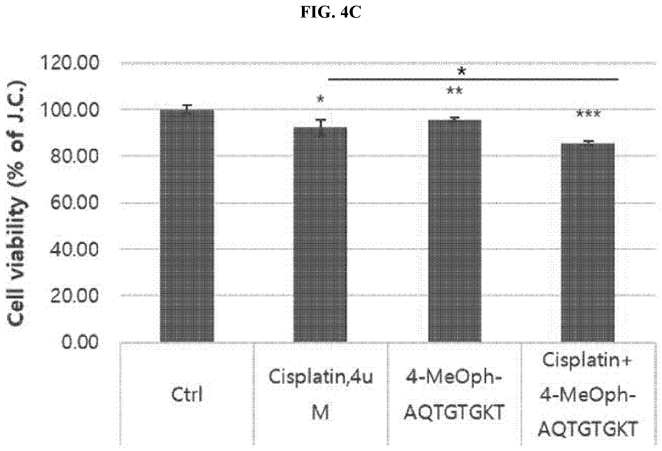

Additionally, HCC1937 cells were treated with 4-MeOph-AQTGTGKT at a concentration of 5 µM and simultaneously the chemotherapeutic agent Cisplatin was administered either alone or in combination at a concentration of 4 µM. After 48 hours, the cell viability inhibitory effect was compared. As shown in FIG. 4C, when 4-MeOph-AQTGTGKT was administered alone, there was a decrease in cell viability of about 4.6% compared to the control group, and when Cisplatin was administered alone, there was a decrease in cell viability of about 7.9% compared to the control group. In contrast, the combined treatment of 4-MeOph-AQTGTGKT and Cisplatin resulted in approximately 15% cell viability inhibitory effect.

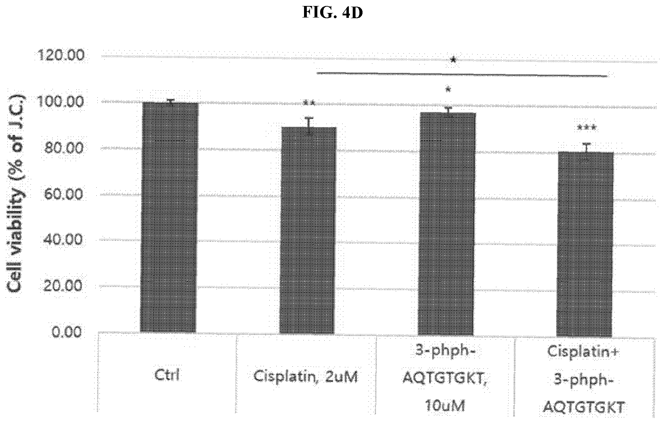

Next, HCC1937 was treated with 3-PhPh-AQTGTGKT at a concentration of 10 µM, and Cisplatin was simultaneously administered either alone or in combination at a concentration of 2 µM. After 72 hours, the cell viability inhibitory effect was compared. As shown in FIG. 4D, when 3-PhPh-AQTGTGKT was administered alone, there was a decrease in cell viability of about 3% compared to the control group, and when Cisplatin was administered alone, there was a decrease in cell viability of about 10% compared to the control group. In contrast, the combined treatment of 3-PhPh-AQTGTGKT and Cisplatin resulted in approximately 20% cell viability inhibitory effect.

### Example 3_{:} Evaluation of Anticancer Effect according to Co-administration of 3-PhPh-AOTGTGKT and Osimertinib

### 3.1. Comparison of Anticancer Effects Depending on Timing of Co-administration with 3-PhPh-AQTGTGKT

To explore the optimal regimen for the combined treatment of Osimertinib and 3-PhPh-AQTGTGKT, an experiment was conducted by dividing it into groups that were treated with Osimertinib alone after co-administration with 3-PhPh-AQTGTGKT and groups that were co-administered with Osimertinib and 3-PhPh-AQTGTGKT after treating with Osimertinib alone in PC9/OR lung cancer cell lines. More specifically, combination 1 (Combo 1) treated with 4 µM Osimertinib and 25 µM 3-PhPh-AQTGTGKT for 3 days, then replaced the medium and treated with Osimertinib alone for 3 days. Combination 2 (Combo 2) treated with 4 µM Osimertinib alone for 3 days, then replaced the medium and treated with 4 µM Osimertinib and 25 µM 3-PhPh-AQTGTGKT for 3 days. The cells were then stained with crystal violet and the cell growth was measured at 570 run with a spectrophotometer.

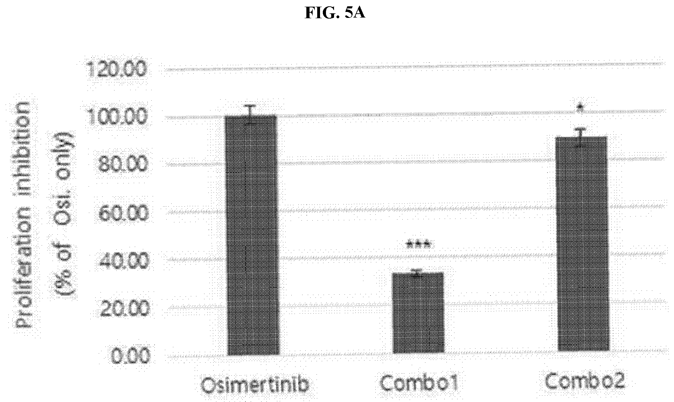

As shown in FIG. 5A, it showed a more remarkable cell growth inhibitory effect in the case of first co-administering Osimertinib and 3-PhPh-AQTGTGKT and then treating Osimertinib alone (Combo 1) than in the case of co-administering Osimertinib and 3-PhPh-AQTGTGKT after treating Osimertinib alone (Combo 2).

### 3.2. Comparison of Anticancer Effects Depending on Duration of Co-administration with 3-PhPh-AQTGTGKT

To further specifically explore the optimal co-administration regimen indicated in Example 3.1, an experiment was conducted by dividing it into groups that were treated with Osimertinib alone after co-administration with Osimertinib and 3-PhPh-AQTGTGKT, and groups that continued co-administration of Osimertinib and 3-PhPh-AQTGTGKT in PC9/OR lung cancer cell lines. More specifically, combination 1 (Combo 1) treated with 1 µM Osimertinib and 25 µM 3-PhPh-AQTGTGKT for 3 days, then replaced the medium and treated with Osimertinib alone for 3 days. Combination 2 (Combo 2) treated with 1 µM Osimertinib and 25 µM 3-PhPh-AQTGTGKT for 3 days, then replaced the medium and repeated the treatment with 1 µM Osimertinib and 25 µM 3-PhPh-AQTGTGKT for 3 days. The cells were then stained with crystal violet and the cell growth was measured at 570 run with a spectrophotometer.

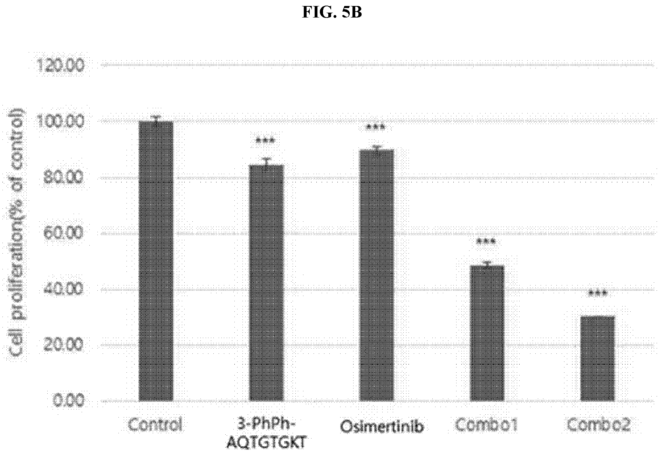

As shown in FIG. 5B, it showed a more remarkable cell growth inhibitory effect in the case of repeating the treatment with 1 µM Osimertinib and 25 µM 3-PhPh-AQTGTGKT for 3 days (Combo 2) than in the case of first co-administering Osimertinib and 3-PhPh-AQTGTGKT and then treating Osimertinib alone (Combo 1).

### Example 4: Anticancer Effect of Co-administration of 3-PhPh-AQTGTGKT and Osimertinib in Lung Cancer Animal Model

Nude mice transplanted with H820 lung cancer cell line, an animal model of lung cancer described in the "Experimental Materials and Methods," was treated 7 times at a two-day interval for 14 days with 3-PhPh-AQTGTGKT at a dose of 10 mpk, and Osimertinib was orally administered daily at 2.5 mpk. Then, the growth of the tumor was compared.

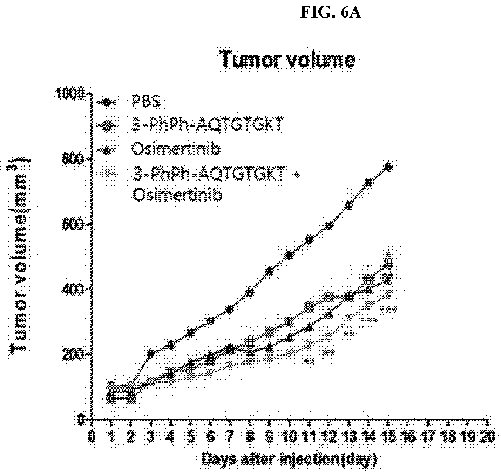

As shown in FIG. 6A, the tumor viability was reduced by about 38% compared to the control group (PBS administered group) when 3-PhPh-AQTGTGKT was administered alone, Osimertinib's single administration reduced the growth by 46%, and the group treated by co-administering 3-PhPh-AQTGTGKT and Osimertinib showed a tumor viability inhibitory effect of 51%.

In addition, the combination efficacy of the compound of the present invention and Osimertinib after the expression of Osimertinib resistance was evaluated in the lung cancer animal model using the H1975 cell line. After inoculating the H1975 cell line in 4-week-old female Balb/C nude mice, the presence and growth of the tumor were observed and the short and long axes of the tumor were measured to calculate the volume. When the volume of the tumor reached 70-120 mm³, the groups were randomly separated and PBS, the negative control group, and Osimertinib at 5 mpk were orally administered daily.

The first day of administration was set as day 1, and the volume of the tumor was measured twice a week. As a result, the PBS group reached 1,000 mm³ on the 20^{th} day of administration, and the group administered with 5 mpk Osimertinib started to decrease depending on the number of administrations from the start day of administration, and it was confirmed that the growth of the tumor was significantly suppressed compared to the PBS group (P<0.001).

However, from the 24^{th} day of Osimertinib administration, it was found that the tumor started to grow again, and when comparing the tumor size of the Osimertinib administration group on the 28^{th} day of administration with the last measured tumor size, the tumor viability rate was over 50%, and it was judged that resistance to Osimertinib was acquired. Accordingly, the individuals who orally administered 5 mpk Osimertinib every day for 28 days were further divided into two groups and observed until the 32^{nd} day of administration. As the tumor gradually increased, the group orally administered 5 mpk Osimertinib daily and intravenously administered PBS at a two-day interval (Osimertinib+PBS group); and the group orally administered 5 mpk Osimertinib daily and intravenously administered 10 mpk 3-PhPh-AQTGTGKT at a two-day interval (Osimertinib+3-PhPh-AQTGTGKT group) were separated for efficacy evaluation.

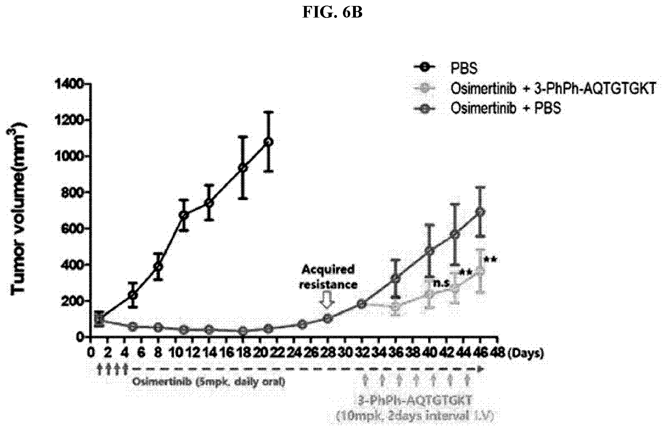

The results are shown in FIG. 6B. From the initial 2 doses after the onset of Osimertinib resistance, the growth of the tumor in the Osimertinib+3-PhPh-AQTGTGKT group was delayed compared to the Osimertinib+PBS group, and statistically significant differences began to appear between the tumor viability curves of the Osimertinib+3-PhPh-AQTGTGKT group and the Osimertinib+PBS group after 7 doses (14 days after administration) (P<0.01). On the day of termination of administration, the size of the tumor was measured, and the Osimertinib+PBS group was measured to be an average of 692.6 mm³, and the size of the tumor in the Osimertinib+3-PhPh-AQTGTGKT group was measured to be 364.7 mm³. It was confirmed that the tumor size in the Osimertinib+3-PhPh-AQTGTGKT administration group was reduced by about 52% compared to the Osimertinib+PBS group. No weight loss or death of any animals was observed during the experiment.

The above results show that co-administration of 3-PhPh-AQTGTGKT and Osimertinib in the heterotransplant lung cancer animal model is much more effective than single administration.

### Example 5: Evaluation of Autophagy Effect due to Co-administration

Nude mice transplanted with H820 lung cancer cell line, an animal model of lung cancer described in the "Experimental Materials and Methods," was treated 7 times at 2-day intervals for 14 days with 3-PhPh-AQTGTGKT at a dose of 10 mpk. Osimertinib was orally administered daily at 2.5 mpk, followed by tumor sampling and p-Beclin1S15 immunostaining.

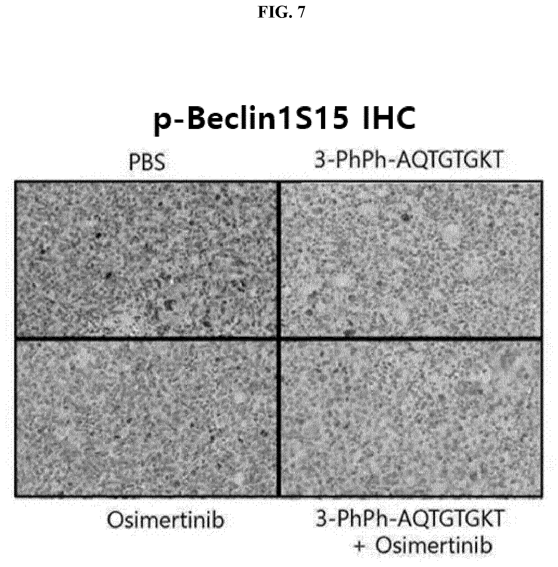

As shown in FIG. 7, in the heterotransplant lung cancer animal model, the expression of p-Beclin1515 was reduced in the group treated solely with 3-PhPh-AQTGTGKT compared to the group receiving Osimertinib alone, and was much more reduced in the co-administration group of 3-PhPh-AQTGTGKT and Osimertinib.

The description of the present invention mentioned above is for illustrative purposes, and one having ordinary skill in the technical field to which the present invention belongs will understand that the technical idea or essential features of the present invention may be easily modified into other specific forms without changing them. Therefore, the examples described above should be considered in all respects as illustrative and not restrictive.

### [Industrial Applicability]

The present invention relates to a pharmaceutical composition for preventing or treating cancer and a composition for enhancing the anticancer effect of an anticancer drug, wherein the compositions comprise oligopeptide AQTGTGKT and analog compounds thereof as active ingredients based on the autophagy mechanism involved in the resistance of conventional anticancer drugs as a tumor promoter. The compounds according to the present invention may selectively bind to a protein target involved in the upper stage of autophagy only when cancer progresses, thereby over-activating autophagy, specifically targeting only cancer cells without affecting normal cells. Therefore, it is possible to maximize the inhibitory effect on cancer cell viability while minimizing the side effects of the effect of single administration of conventional anticancer drugs in patients resistant to anticancer drugs.

More specifically, when oligopeptide AQTGTGKT and its analog compounds are co-administered with conventional anticancer drugs, they demonstrate an enhanced anticancer effect and may significantly suppress cancer cell viability with excellent anticancer effects even with low concentrations of the drug. This may minimize side effects such as functional and active damage appearing in normal tissues, bone marrow dysfunction, gastrointestinal disturbance, alopecia, and anticancer drug resistance. In addition, oligopeptides have the advantage of being small in molecular weight compared to antibodies, reducing the concern of immune responses, and easy penetration into tissues. Therefore, it is anticipated that oligopeptides may be utilized in combination with anticancer drugs for various types of cancer.

## Claims

1. A pharmaceutical composition for preventing or treating cancer, comprising (i) a compound represented by the following General Formula; and (ii) an anticancer drug as an active ingredient:
[General Formula] **X-AQTGTGKT**
(In the General Formula, A represents alanine, Q represents glutamine, T represents threonine, G represents glycine, K represents lysine, and
X is absent; or at least one selected from the group consisting of

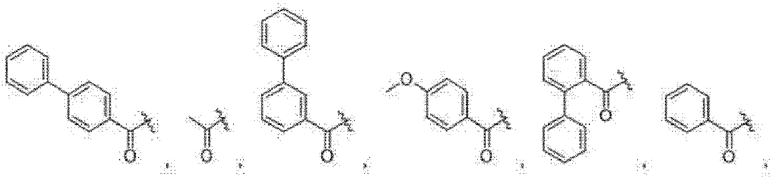

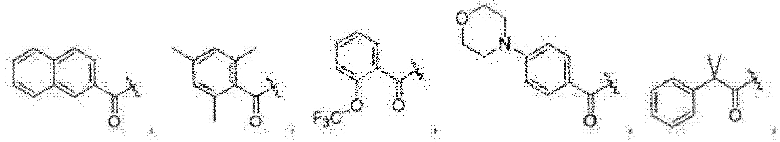

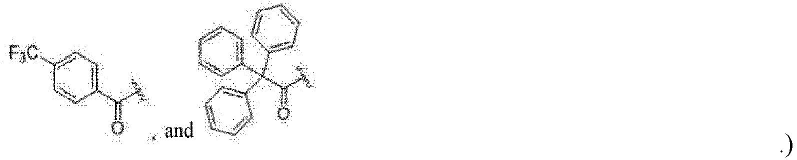

2. The pharmaceutical composition of claim 1, wherein the X is absent; or at least one selected from the group consisting of

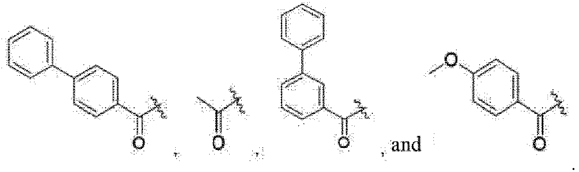

3. The pharmaceutical composition of claim 1, wherein the anticancer drug is at least one selected from the group consisting of targeted anticancer drugs and chemotherapeutic agents.

4. The pharmaceutical composition of claim 3, wherein the targeted anticancer drug is at least one selected from the group consisting of tyrosine kinase inhibitor, PARP inhibitor, angiogenesis inhibitor, CDK4/6 inhibitor, hormonal therapy drug, and antibody-drug conjugate.

5. The pharmaceutical composition of claim 4, wherein the tyrosine kinase inhibitor is a targeted drug for at least one selected from the group consisting of epidermal growth factor receptor (EGFR), anaplastic lymphoma kinase (ALK), ROS Proto-Oncogene 1 (ROS1), B-Raf Proto-Oncogene (BRAF), human epidermal growth factor receptor 2 (HER2), Ret Proto-Oncogene (RET), Neurotrophic Receptor Tyrosine Kinase 1 (NTRK1), Mesenchymal-Epithelial Transition factor (MET), and Neuregulin 1 (NRG1).

6. The pharmaceutical composition of claim 4, wherein the tyrosine kinase inhibitor is at least one selected from the group consisting of Osimertinib, Afatinib, Brigatinib, Dasatinib, Dacomitinib, Erlotinib, Gefitinib, Lapatinib, Neratinib, Vandetanib, Icotinib, Varitinib, Tesevatinib, Canertinib, Naquotinib, Pelitinib, Poziotinib, Rociletinib, Nazartinib, Allitinib (ALS-1306), Pyrotinib, Tyrphostin, Crizotinib, Ceritinib, Entrectinib, Dabrafenib, Trametinib, Alectinib, Lorlatinib, Larotectinib, Lasertinib, Olmutinib, AG 1478, CUDC-101, MTKi-327 (JNJ-26483327), CL-387785 (EKI-785), CNX-2006, PD168393, TAK285, WZ4002, and AV-412 (MP-412).

7. The pharmaceutical composition of claim 4, wherein the PARP inhibitor is at least one selected from the group consisting of Olaparib, Rucaparib, Talazoparib, Veliparib, and Niraparib.

8. The pharmaceutical composition of claim 4, wherein the CDK4/6 inhibitor is at least one selected from the group consisting of Trilaciclib, Palbociclib, Ribociclib, and Abemaciclib.

9. The pharmaceutical composition of claim 4, wherein the hormonal therapy drugs is at least one selected from the group consisting of Tamoxifen, Toremifene, Fulvestrant, Goserelin, Leuprolide, Anastrozole, Letrozole, and Exemestane.

10. The pharmaceutical composition of claim 4, wherein the antibody-drug conjugate is at least one selected from the group consisting of Sacituzumab govitecan and Ladiratuzumab.

11. The pharmaceutical composition of claim 3, wherein the targeted anticancer drug is at least one selected from the group consisting of Daratumumab, Trastuzumab, and Rituximab.

12. The pharmaceutical composition of claim 3, wherein the chemotherapeutic agent is at least one selected from the group consisting of Alimta, Oxaliplatin, Pemetrexed, Cisplatin, Gemcitabine, Carboplatin, 5-Fluorouracil (5-FU), Cyclophosphamide, Paclitaxel, Vincristine, Etoposide, and Doxorubicin.

13. The pharmaceutical composition of claim 1, wherein the compound enhances the anticancer effect of the anticancer drug and reduces side effects.

14. The pharmaceutical composition of claim 1, wherein the composition is a mixture form in which the compound and the anticancer drug are mixed.

15. The pharmaceutical composition of claim 1, wherein the composition is a form in which the compound and the anticancer drug are each formulated and administered simultaneously or sequentially.

16. The pharmaceutical composition of claim 1, wherein the anticancer drug is comprised at a concentration of 0.1 to 10 µM relative to the total composition.

17. The pharmaceutical composition of claim 1, wherein the compound represented by the General Formula is comprised at a concentration of 1 to 50 µM relative to the total composition.

18. The pharmaceutical composition of claim 1, wherein the anticancer drug is a targeted anticancer drug, and X in the compound represented by the General Formula is absent; or at least one selected from the group consisting of

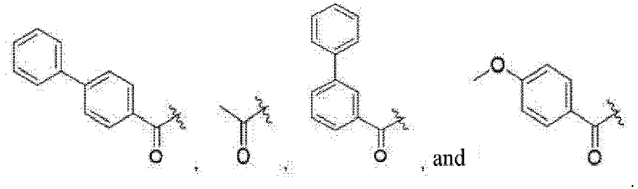

19. The pharmaceutical composition of claim 18, wherein the targeted anticancer drugs and the compound are comprised at a molarity ratio of 1:1 to 1:500.

20. The pharmaceutical composition of claim 1, wherein the anticancer drugs is a chemotherapeutic agent, and X in the compound represented by the General Formula is at least one selected from the group consisting of

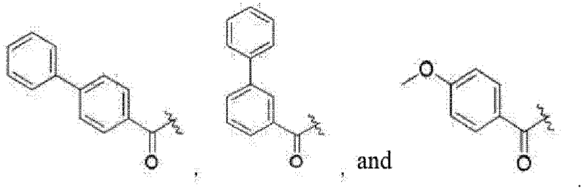

21. The pharmaceutical composition of claim 20, wherein the chemotherapeutic agent and the compound is comprised at a molarity ratio of 1:1 to 1:500.

22. The pharmaceutical composition of claim 1, wherein the cancer is a cancer selected from the group consisting of lung cancer, breast cancer, blood cancer, colorectal cancer, pancreatic cancer, and combinations thereof.

23. A kit for preventing or treating cancer comprising (i) a compound represented by the following General Formula; and (ii) an anticancer drug as an active ingredient:
[General Formula] X-AQTGTGKT
(In the General Formula, A represents alanine, Q represents glutamine, T represents threonine, G represents glycine, K represents lysine, and
X is absent; or at least one selected from the group consisting of 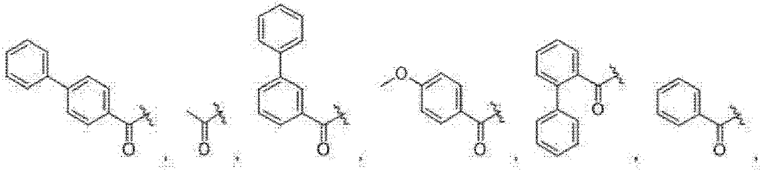 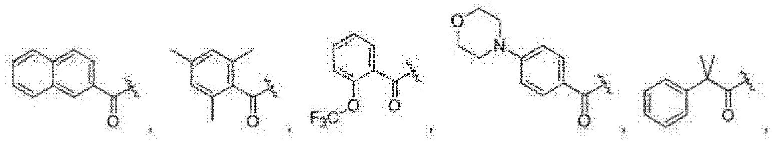 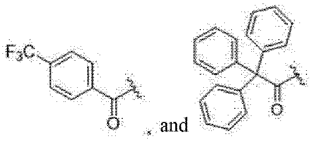 .)

24. A pharmaceutical composition for enhancing the anticancer effect of an anticancer drug, comprising the compound represented by the following General Formula as an active ingredient:
[General Formula] X-AQTGTGKT
(In the General Formula, A is alanine, Q is glutamine, T is threonine, G is glycine, K is lysine,
X is absent; or at least one selected from the group consisting of

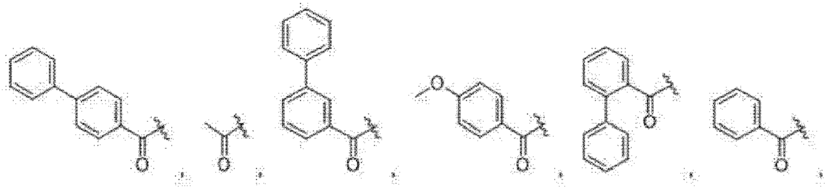

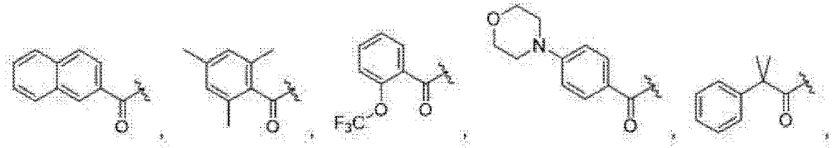

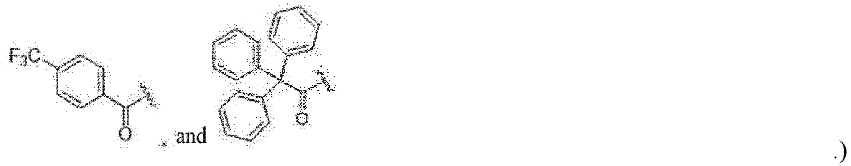

25. The pharmaceutical composition of claim 24, wherein the composition is administered simultaneously, separately, or sequentially with an anticancer drug.

26. The pharmaceutical composition of claim 24, wherein the cancer is a cancer selected from the group consisting of lung cancer, breast cancer, blood cancer, colorectal cancer, pancreatic cancer, and combinations thereof.

27. A method for preventing or treating cancer, comprising the step of administering the composition of claim 1 to a subject in need thereof.

28. A use of the composition of claim 1 for manufacturing a drug for treating cancer.

29. A use of the composition of claim 1 for preventing or treating cancer.
